# FASCICULE DE BREVET EUROPEEN

(11) **EP 2 321 247 B1**
(45) Date de publication et mention de la délivrance du brevet: **17.02.2016**
(21) Numéro de dépôt: 09797516.3
(22) Date de dépôt: 17.07.2009
(51) Int. Cl.: C07C 45/81, C07C 47/58, C07C 47/575, B01D 1/06

(54) **PROCEDE DE SEPARATION D'UN ALDEHYDE AROMATIQUE**
VERFAHREN ZUR TRENNUNG VON AROMATISCHEM ALDEHYD
METHOD FOR SEPARATING AROMATIC ALDEHYDE

(30) Priorité: 18.07.2008 FR 0804103
(43) Date de publication de la demande: 18.05.2011
(73) Titulaire: Rhodia Opérations, 75009 Paris (FR)
(72) Inventeur: GAYET, Hubert, F-69100 Villeurbanne (FR); MASSON, Jean-Claude, F-69003 Lyon (FR)
(74) Mandataire: Dutruc-Rosset, Marie-Claude
(86) Numéro de dépôt international: PCT/EP2009/059226
(87) Numéro de publication internationale: WO 2010/007161

(56) Documents cités:
- WO-A-2004/063140
- DE-A1- 2 930 222
- FR-A- 1 040 559
- FR-A- 1 043 070
- FR-A1- 2 253 542
- GB-A- 401 562
- US-A- 5 294 406
- US-A- 6 562 996

## Description

La présente invention a pour objet un procédé de séparation d'un aldéhyde aromatique à partir d'un mélange comprenant en outre des goudrons.

Plus particulièrement, l'invention concerne la séparation d'un aldéhyde hydroxy- et/ou alkoxy-aromatique.

L'invention vise plus particulièrement le traitement des goudrons issus d'un procédé de préparation et de purification de la vanilline (4-hydroxy-3-méthoxybenzaldéhyde) et de l'éthylvanilline (3-éthoxy-4-hydroxybenzaldéhyde).

Les aldéhydes hydroxy- et/ou alkoxyaromatiques sont des produits très importants utilisés d'abord comme arômes et parfums puis ensuite comme produits intermédiaires dans de nombreux domaines tels que par exemple, l'agrochimie, la pharmacie, la cosmétique et autres industries.

En particulier, la vanilline et l'éthylvanilline sont des produits essentiellement à vocation alimentaire. Il est donc important de proposer sur le marché des produits de haute pureté chimique et de bonne qualité gustative.

Ainsi, leur procédé de synthèse nécessite des opérations de séparation et de purification poussée.

Il a été proposé différents procédés pour la synthèse des aldéhydes aromatiques.

Les procédés les plus importants sont basés sur la fonctionnalisation d'un composé phénolique de départ, par exemple, phénol, catéchol, dérivé du catéchol, gaïacol (ou 2-méthoxyphénol), guétol (ou 2-éthoxyphénol).

Intervient, généralement, dans ce type de procédé, le composé phénolique sous une forme salifiée, par exemple, sous forme d'un sel de sodium : un groupe formyle est additionné en position para du groupe hydroxyle présent sur le cycle benzénique, selon différentes méthodes.

On a proposé selon EP-A-0 773 919, un procédé de préparation notamment de la vanilline qui consiste à faire réagir le formol et le gaïacol, en présence de soude conduisant à un mélange comprenant l'ohydroxyméthylgaïacol (OMG), le p-hydroxyméthylgaïacol (PMG), le 4,6-di(hydroxyméthyl)gaïacol (DMG), puis à oxyder ledit mélange par l'oxygène en présence d'un catalyseur au palladium et d'un catalyseur au bismuth et ensuite à éliminer dans les produits d'oxydation le contenant, le groupe carboxylique situé en position ortho, permettant ainsi d'obtenir la vanilline avec un bon rendement réactionnel.

Par ailleurs, il existe une voie d'accès à la vanilline totalement différente qui consiste à faire réagir en milieu basique, le gaïacol et l'acide glyoxylique conduisant à l'acide 4-hydroxy-3-méthoxymandélique puis à oxyder le condensat obtenu.

A l'issue de la réaction d'oxydation, on obtient le précurseur de la vanilline c'est-à-dire le groupe hydroxyle est sous forme salifiée, de préférence sous forme sodée ; l'excès de gaïacol également sous forme salifiée et diverses impuretés.

Dans une étape suivante, on effectue la libération de la vanilline et du gaïacol présents dans le milieu réactionnel par acidification effectuée à l'aide d'un acide fort, par exemple l'acide sulfurique.

Pour séparer la vanilline du mélange brut réactionnel, une méthode connue consiste à effectuer son extraction à l'aide d'un solvant organique.

Pour isoler la vanilline du solvant d'extraction, on effectue une distillation dudit mélange permettant d'obtenir en tête de distillation le solvant d'extraction (qui est le composé le plus volatil du mélange), et en pied de distillation, une "vanilline brute", à savoir un mélange comprenant essentiellement de la vanilline, associée à des impuretés lourdes dénommées « goudrons » et à de faibles quantités d'impuretés légères.

Le brevet FR 2 253 542 décrit par ailleurs un procédé de séparation de dérivés de vanilline par évaporation à partir de solution faiblement concentrée en aldéhyde et qui utilise un évaporateur à film tombant.

Le problème est donc de récupérer le produit noble à savoir l'aldéhyde aromatique présent dans les goudrons selon un procédé qui soit à la fois respectueux de l'intégrité des produits à séparer tout en étant économique et facile à mettre en oeuvre dans des dispositifs industriels tout particulièrement dans ceux qui fonctionnent en continu.

La présente invention concerne un procédé de séparation et de récupération d'un aldéhyde aromatique à partir d'un mélange comprenant un aldéhyde aromatique (défini ci-après), des impuretés légères et lourdes, des goudrons caractérisé par le fait que l'on introduit le flux liquide comprenant ledit mélange dans un évaporateur, que l'on vaporise ledit mélange de telle sorte que l'on sépare d'une part un flux gazeux comprenant l'aldéhyde aromatique, les impuretés légères, les impuretés lourdes vaporisables à la température d'évaporation et de pression choisies et d'autre part un flux liquide comprenant essentiellement les goudrons et que l'on récupère l'aldéhyde aromatique à partir du flux gazeux séparé.

Une étape de concentration du mélange est effectuée avant l'étape de séparation par évaporation, la concentration de l'aldéhyde aromatique dans le flux liquide après l'étape de concentration étant compris entre 10% et 60% en poids et celle des lourds entre 40% et 90% en poids.

Selon une variante du procédé de l'invention, on recycle le flux gazeux séparé comprenant essentiellement l'aldéhyde aromatique obtenu, à l'état gazeux ou après condensation, dans une étape en amont de cette étape de séparation.

L'invention concerne un procédé de séparation et de récupération de l'aldéhyde aromatique à partir d'un mélange comprenant un aldéhyde aromatique, des impuretés légères et lourdes et des goudrons caractérisé par le fait que l'on effectue au préalable une concentration du flux à introduire dans l'évaporateur.

Ainsi, on effectue avant cette étape de séparation, une concentration des goudrons et des impuretés lourdes par distillation permettant de récupérer en tête de distillation l'aldéhyde aromatique et les impuretés légères et en pied de distillation, les goudrons et les impuretés lourdes.

Selon une variante préférée du procédé de l'invention, on recycle le flux gazeux séparé comprenant essentiellement l'aldéhyde aromatique obtenu, à l'état gazeux ou après condensation, à l'étape de concentration.

Dans le présent texte, on entend par « impuretés légères » des composés dont la volatilité relative est plus élevée que celle de l'aldéhyde aromatique, dans les conditions de température et de pression considérées.

Les impuretés légères sont le composé phénolique de départ par exemple, gaïacol, guétol ; les isomères éventuels de l'aldéhyde aromatique comme l'o-vanilline et l'o-éthylvanilline ; des traces du solvant d'extraction et des impuretés éventuelles présentes dans le solvant et les réactifs de départ.

Par «lourds » et « impuretés lourdes », on désigne des composés dont la volatilité relative est plus faible que celle de l'aldéhyde aromatique, dans les conditions de température et de pression considérées.

Les « lourds » sont essentiellement les goudrons qui résultent des étapes réactionnelles précédentes et également en quantités moindres, les impuretés lourdes du procédé comme par exemple, celles qui résultent de l'oxydation du groupe formyle en groupe carboxylique.

Le mélange de départ comprend généralement de 55 à 94 % en poids d'un aldéhyde aromatique, de 6 à 45% en poids des impuretés légères et lourdes, des goudrons.

La composition du mélange est donnée à titre indicatif et le procédé de l'invention convient également pour des mélanges plus riches en aldéhyde aromatique (par exemple une teneur supérieure à 99 % en poids en aldéhyde aromatique) ou plus riches en impuretés (par exemple une teneur supérieure à 60 % en poids).

Typiquement, une vanilline brute en sortie de distillation du solvant d'extraction comprend de 55 % à 94 % en poids de vanilline, incluant les impuretés légères qui représentent de 1 à 10 % du poids de la vanillline et les lourds à raison de 5 à 60 % en poids.

Le procédé de l'invention s'applique tout particulièrement à la vanilline et à l'éthylvanilline mais il convient également pour d'autres aldéhydes aromatiques.

Par « aldéhyde aromatique », on entend un composé de type benzaldéhyde substitué ou non, c'est-à-dire plus précisément un noyau benzénique comprenant au moins un groupe formyle.

Il répond à la formule suivante: 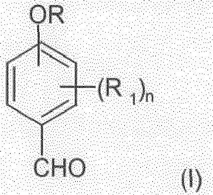 dans ladite formule :
- R représente un atome d'hydrogène ou un groupe alkyle,
- les groupes R₁, identiques ou différents, représentent un atome d'hydrogène, un groupe hydroxyle, un groupe alkyle ou alkoxy,
- n est un nombre de 0 à 4.

Dans le cadre de l'invention, on entend par « alkyle », une chaîne hydrocarbonée linéaire ou ramifiée ayant de 1 à 12 atomes de carbone et de préférence de 1 ou 4 atomes de carbone. Des exemples préférés de groupes alkyle sont les groupes méthyle, éthyle, propyle, isopropyle.

Par « alkoxy », on entend un groupe alkyl-O- dans lequel le terme alkyle a la signification donnée ci-dessus. Des exemples préférés de groupes alkoxy sont les groupes méthoxy ou éthoxy.

L'aldéhyde aromatique mis en oeuvre préférentiellement dans le procédé de l'invention répond à la formule (I) dans laquelle R représente un atome d'hydrogène ou un groupe méthyle ou éthyle et R₁ représente un atome d'hydrogène ou un groupe méthoxy ou éthoxy.

Dans la formule (I), n est de préférence égal à 1 ou 2.

Comme exemples plus spécifiques d'aldéhydes aromatiques, on peut citer notamment :
- la vanilline,
- l'éthylvanilline (3-éthoxy-4-hydroxybenzaldéhyde),
- l'isovanilline (4-méthoxy-3-hydroxybenzaldéhyde),
- l'o-vanilline (3-méthoxy-2-hydroxybenzaldéhyde),
- l'aldéhyde protocatéchique,
- l'aldéhyde syringique (3,5-diméthoxy-4-hydroxybenzaldéhyde),
- l'aldéhyde vératrique (3,4-diméthoxybenzaldéhyde),
- le p-hydroxybenzaldéhyde,

Le procédé de l'invention s'applique sur un milieu réactionnel comprenant au moins un aldéhyde aromatique et diverses impuretés, ledit aldéhyde étant extrait, après introduction d'un groupe formyle, par un solvant organique.

Ainsi, en fin de réaction, l'aldéhyde aromatique est extrait avec un solvant organique ou un mélange de solvants organiques.

On fait appel à un solvant organique qui soit inerte par rapport à l'aldéhyde aromatique.

Comme solvants susceptibles d'être utilisés, on peut citer notamment les hydrocarbures aliphatiques, cycloaliphatiques, aromatiques, halogénés ou non ; les alcools ; les cétones et les nitriles.

On mentionne plus particulièrement comme hydrocarbures aliphatiques, cycloaliphatiques ou aromatiques, l'heptane, le cyclohexane, le méthylcyclohexane, le benzène, le toluène ; comme hydrocarbures aliphatiques, cycloaliphatiques ou aromatiques halogénés, le dichlorométhane, le trichlorométhane, le dichloroéthane, le chlorobenzène, les dichlorobenzènes ; comme alcools, le méthanol, l'éthanol, le propanol, l'isopropanol, le butanol ; comme cétones, l'acétone, la méthyléthylcétone, la méthylisobutylcétone, la diisobutylcétone ; comme nitriles, l'acétonitrile.

On peut mettre en oeuvre un mélange desdits solvants.

L'opération d'extraction est effectuée à une température qui dépend de la nature du solvant.

La température est choisie avantageusement entre 0°C et 90°C, de préférence entre 20°C et 80°C.

Pour isoler l'aldéhyde aromatique, on commence par éliminer le solvant d'extraction, par une opération de distillation.

Dans tout le procédé de l'invention, les flux sont maintenus en température par des systèmes de double enveloppe avec circulation de caloporteur ou de vapeur d'eau.

Comme mentionné précédemment, l'aldéhyde aromatique présent dans le milieu réactionnel est extrait à l'aide d'un solvant organique dont des exemples sont mentionnés ci-dessus.

En fin d'opération d'extraction, on obtient un flux liquide (*F₁*) comprenant l'aldéhyde aromatique en solution dans un solvant d'extraction. Il comprend la plus souvent de l'aldéhyde aromatique à une concentration variant entre 5 et 70 % en poids, de 20 % à 94,8 % en poids d'un solvant organique et différentes impuretés légères et des lourds à une concentration variant entre 0,2 et 10 % en poids.

Le flux liquide (*F₁*) comprend plus préférentiellement de 10 à 60 % en poids d'aldéhyde aromatique, de 35 % à 89 % en poids d'un solvant organique et de 1 à 5 % en poids des différentes impuretés légères et des lourds.

Une première étape consiste donc à effectuer la séparation de l'aldéhyde aromatique et du solvant d'extraction par une opération de distillation qui assure sa séparation.

Plus précisément, la distillation est conçue pour obtenir :
- en pied de distillation, une phase liquide (*F₅*) comprenant l'aldéhyde aromatique accompagné de différentes impuretés légères et des lourds,
- en tête de distillation, une phase gazeuse (*F₂*) comprenant le solvant d'extraction et de l'aldéhyde aromatique à une faible concentration permettant le recyclage du solvant.

Par « faible concentration », on entend une concentration d'aldéhyde aromatique de préférence d'au plus 100 ppm (0,01 % en poids) dans le solvant organique.

L'étape de distillation est conduite à une température et une pression qui dépend du solvant d'extraction.

Généralement, elle se situe par exemple pour la vanilline, en pied de distillation entre 150°C et 190°C, de préférence entre 160°C et 180°C.

La distillation est conduite de préférence sous pression réduite établie en tête de colonne.

La pression réduite définie en pied de distillation est comprise généralement entre 20 et 100 mm de mercure, de préférence entre 20 et 40 mm de mercure.

Selon une variante préférée du procédé de l'invention, on conduit cette opération sous atmosphère de gaz inerte, de préférence sous atmosphère d'azote.

L'opération de distillation est effectuée dans une installation de distillation classique, par exemple une ou plusieurs colonnes de distillation en série.

Cette étape de distillation vise à obtenir en pied l'aldéhyde aromatique (*F₅*) dépourvu du solvant organique.

L'homme du métier est parfaitement en mesure de choisir les moyens à mettre en oeuvre en fonction de la séparation à effectuer.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et les puretés des produits devant être obtenues en tête et en pied de distillation.

On précisera que la colonne peut être garnie indifféremment de plateaux ou de garnissage ordonné ou tissé, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 20, de préférence de 2 à 10,
- taux de reflux R compris entre 0,05 et 15, de préférence entre 0,1 et 3.

Le taux de reflux est défini par le rapport du débit de matière réinjectée de la tête de la colonne vers l'intérieur de la colonne et du débit sortant effectivement à la sortie de la tête de colonne.

Pour effectuer la distillation, l'apport des calories en pied de colonne peut être fait notamment par un échangeur tubes calandre chauffé à la vapeur ou par un fluide caloporteur, par l'intermédiaire de serpentins de chauffage alimentés à la vapeur ou par un fluide caloporteur ou par tout autre dispositif équivalent.

Un mode de réalisation préféré consiste à chauffer le mélange en pied de distillation dans un échangeur thermique par prélèvement d'un flux *(F₆)* en pied qui circule en boucle. Plus précisément, le flux *(F₅)* sort en pied de distillation et une fraction *(F₆)* traverse de bas en haut, un échangeur thermique et en sortie d'échangeur est introduite latéralement dans la partie inférieure de la colonne de distillation.

Un autre mode de réalisation consiste à effectuer une circulation forcée du flux *(F₅)* dans l'échangeur à l'aide d'une pompe.

Le flux gazeux *(F₂)*, en tête de colonne, comprenant majoritairement le solvant organique et l'aldéhyde aromatique à une concentration de préférence d'au plus de 100 ppm, est condensé de manière à récupérer un flux liquide dont une fraction *(F₃)* est introduite, latéralement en tête de colonne, pour assurer le reflux dans la colonne et l'autre fraction *(F₄)* peut être recyclée à l'étape d'extraction.

La récupération du solvant organique à partir du flux *(F₂)* se fait par condensation.

On refroidit la phase gazeuse *(F₂)* et la transforme sous forme liquide par refroidissement en abaissant la température du flux *(F₂)* par exemple, de 15°C à 30°C, par passage dans un ou plusieurs condenseurs de préférence un.

Cette opération est conduite par passage dans un condenseur qui est un appareil classique par exemple un échangeur tubulaire alimenté par de l'eau ou par un fluide (généralement une huile, un alcool par exemple un glycol ou un mélange eau/glycol, une saumure) maintenu à une température voisine de la température de refroidissement choisie, en sortie de condenseur.

Le nombre de condenseurs est choisi en fonction des capacités réfrigérantes des liquides de refroidissement circulant dans les condenseurs.

Dans le cas de condenseurs en série, la phase gazeuse en sortie du premier condenseur est introduite dans le deuxième condenseur.

On récupère à la base du ou des condenseurs, le solvant organique sous forme liquide.

On récupère en pied de distillation un flux *(F₅)* à savoir un "aldéhyde aromatique brut" qui est un mélange comprenant essentiellement de l'aldéhyde aromatique associé à des lourds, impuretés lourdes et « goudrons » et des impuretés légères.

On précise à titre indicatif, que le flux *(F₅)* comprend entre 89 et 98,5 % en poids d'aldéhyde aromatique, entre 1 et 5 % en poids d'impuretés légères et des lourds entre à raison de 0,5 à 6 % en poids.

Comme mentionné ci-dessus, une fraction *(F₆)* est recyclée sur la colonne de distillation et l'autre fraction *(F₇)* est soumise aux opérations suivantes notamment de concentration.

Selon une variante préférée du procédé de l'invention, il a été trouvé que l'aldéhyde aromatique obtenu était d'une meilleure qualité dès lors que l'on effectuait avant l'étape de séparation par évaporation, une étape de concentration du mélange à traiter.

Selon une variante préférée du procédé de l'invention, on effectue une concentration du flux *(F₇)* afin d'augmenter la concentration des lourds dans le flux qui est envoyé dans l'évaporateur.

La concentration du flux *(F₇)* est effectuée selon une opération de distillation.

Plus précisément, la distillation est conçue pour obtenir :
- en pied de distillation, *(F₁₁)*, l'aldéhyde aromatique accompagné des lourds,
- en tête de distillation, une phase gazeuse *(F₈)* comprenant essentiellement l'aldéhyde aromatique, les impuretés légères et des impuretés lourdes à une teneur inférieure à 100 ppm (0,01 % en poids), de préférence inférieure à 50 ppm (0,005 % en poids).

L'étape de distillation est conduite à une température en pied de distillation qui se situe entre 160°C et 190°C, de préférence entre 165°C et 185°C.

La distillation est conduite de préférence sous pression réduite établie en tête de colonne. La pression réduite définie en pied de distillation est comprise généralement entre 1 et 25 mm de mercure, de préférence entre 2 et 15 mm de mercure.

Selon une variante préférée de l'invention, on conduit cette opération de distillation, sous atmosphère de gaz inerte, de préférence sous atmosphère d'azote.

L'opération de distillation est effectuée dans un appareil de distillation classique.

Cette étape vise à obtenir en pied un flux liquide (*F₁₁*) comprenant essentiellement l'aldéhyde aromatique ainsi que des impuretés lourdes et des goudrons.

Ainsi, la colonne de distillation pourra être avantageusement, mais non limitativement, une colonne ayant les spécifications suivantes :
- nombre d'étages théoriques : de 1 à 30, de préférence de 6 à 15,
- taux de reflux R compris entre 0,05 et 15, de préférence entre 0,1 et 5.

Pour effectuer la distillation, l'apport des calories en pied de colonne peut être fait notamment par un échangeur tubes calandre chauffé à la vapeur ou par un fluide caloporteur, par l'intermédiaire de serpentins de chauffage alimentés à la vapeur ou par un fluide caloporteur ou par tout autre dispositif équivalent.

Un mode de réalisation préféré consiste à chauffer le mélange en pied de distillation dans un échangeur thermique par prélèvement d'un flux *(F₁₂)* en pied qui circule en boucle. Plus précisément, le flux *(F₁₁)* sort en pied de distillation et une fraction *(F₁₂)* traverse de bas en haut, un échangeur thermique et en sortie d'échangeur est introduite latéralement dans la partie inférieure de la colonne de distillation.

Un autre mode de réalisation consiste à effectuer une circulation forcée du flux *(F₁₁)* dans l'échangeur à l'aide d'une pompe.

Le flux gazeux *(F₈)*, en tête de colonne, comprenant majoritairement l'aldéhyde aromatique et les impuretés légères, est condensé de manière à récupérer un flux liquide dont une fraction *(F₉)* est introduite, latéralement en tête de colonne, pour assurer le reflux dans la colonne et l'autre fraction *(F₁₀)* peut être traitée dans une étape ultérieure de purification de l'aldéhyde aromatique.

En tête de distillation, la récupération du flux comprenant essentiellement l'aldéhyde aromatique à partir du flux *(F₈)* se fait par condensation.

On refroidit la phase gazeuse *(F₈)* et la transforme sous forme liquide par refroidissement en abaissant sa température par exemple de 15°C à 30°C, par passage dans un ou plusieurs condenseurs de préférence un.

Cette opération est conduite par passage dans un condenseur qui est un appareil classique par exemple un échangeur tubulaire alimenté par un fluide caloporteur (généralement une huile) maintenu à une température voisine de la température de refroidissement choisie.

Le nombre de condenseurs est choisi en fonction des capacités réfrigérantes des liquides de refroidissement circulant dans les condenseurs.

Dans le cas de condenseurs en série, la phase gazeuse en sortie du premier condenseur est introduite dans le deuxième condenseur.

On récupère en sortie du ou des condenseurs, l'aldéhyde aromatique *(F₁₀).*

On récupère en pied de distillation un flux *(F₁₁)* à savoir un mélange enrichi en impuretés lourdes et goudrons comprenant essentiellement de l'aldéhyde aromatique associé à des impuretés lourdes et des goudrons.

La concentration de l'aldéhyde aromatique dans ce flux varie entre 10 % et 60 % en poids et celle des lourds varie entre 40 % et 90 % en poids.

Comme mentionné ci-dessus, une fraction *(F₁₂)* est recyclée sur la colonne de distillation et l'autre fraction (*F₁₃*) est soumise à une opération permettant de récupérer l'aldéhyde aromatique présent dans les lourds.

Conformément au procédé de l'invention, on sépare et récupère l'aldéhyde aromatique à partir d'un mélange *(F₁₃)* ayant la même composition que le flux *(F₁₁)* comprenant un aldéhyde aromatique, des lourds caractérisé par le fait que l'on introduit le flux liquide *(F₁₃)* dans un évaporateur, que l'on vaporise ledit mélange de telle sorte que l'on récupère d'une part un flux gazeux *(F₁₄)* comprenant l'aldéhyde aromatique, les impuretés lourdes vaporisables à la température d'évaporation et de pression choisies et d'autre part un flux liquide *(F₁₅)* comprenant essentiellement les impuretés lourdes et les goudrons et que l'on récupère l'aldéhyde aromatique à partir du flux gazeux séparé *(F₁₄).*

Selon une variante du procédé de l'invention, on recycle le flux gazeux obtenu *(F₁₄)*, à l'état gazeux ou après condensation, dans une étape en amont de l'étape de séparation.

Dans une variante préférée du procédé de l'invention, on recycle le flux gazeux *(F₁₄)* séparé, à l'état gazeux ou après condensation, à l'étape de concentration.

Les paramètres de procédé varient selon le type d'évaporateur utilisé dans le procédé de l'invention.

Différents types d'évaporateurs peuvent être choisis conduisant ainsi à plusieurs variantes de procédé.

Conviennent pour la mise en oeuvre du procédé de l'invention, des évaporateurs de plusieurs types, notamment des évaporateur à film tombant ou à film ruisselant, des évaporateurs à film râclé, des évaporateurs à court-trajet à condenseur interne, des évaporateurs râclés horizontaux ou toute variante de ces appareils.

Selon un mode préféré de mise en oeuvre du procédé de l'invention, on conduit la séparation dans un évaporateur maintenu sous atmosphère inerte, de préférence sous atmosphère d'azote.

Ainsi, conformément à une première variante du procédé de l'invention, on effectue la séparation dans un évaporateur à film tombant ou à film ruisselant.

On entend par évaporateur à film tombant (dénommé également évaporateur à film ruisselant), un appareil constitué par une chambre généralement cylindrique comprenant un faisceau de tubes verticaux ; lesdits tubes étant chauffés extérieurement par exemple par circulation de vapeur dans ladite chambre.

Le mélange *(F₁₃)*, comprenant un aldéhyde aromatique, des impuretés lourdes et des goudrons, est alimenté par la partie supérieure et tombe sur un système de distribution par exemple avec déversoir permettant une bonne répartition du liquide au sommet des tubes pour constituer un film liquide d'une fine épaisseur, généralement inférieur à 1 mm, de préférence compris entre 0,3 et 0,5 mm.

Ledit mélange s'écoule par gravité le long de la surface interne des tubes et les fractions vaporisables (dont l'aldéhyde aromatique) sont évaporées au cours de leur cheminement le long des tubes et la phase liquide essentiellement les lourds parvenant à la base des tubes est ensuite séparée des fractions vaporisables.

Le mélange *(F₁₃)* est introduit dans l'évaporateur à film tombant ou à film ruisselant, le traverse de haut en bas et en sortie traverse une enceinte de séparation qui assure la séparation des deux flux liquide et gazeux. Le séparateur peut être un appareil cylindrique ne comprenant aucun interne, c'est-à-dire ni plateaux, ni garnissage.

On récupère en tête de l'enceinte de séparation, un flux gazeux *(F₁₄)* comprenant essentiellement l'aldéhyde aromatique et des impuretés lourdes vaporisables et, en pied d'enceinte de séparation, un flux liquide *(F₁₅)* comprenant essentiellement des lourds, impuretés lourdes et goudrons.

Selon la variante préférée du procédé de l'invention, le flux gazeux *(F₁₄)* est recyclé en amont de cette étape de séparation par évaporation, et plus particulièrement à de l'étape de concentration.

Généralement, ce flux est introduit latéralement sur la colonne de concentration : ce flux pouvant être éventuellement condensé afin d'être introduit sous forme liquide par l'intermédiaire d'une pompe.

Dans le flux gazeux *(F₁₄)*, la concentration en aldéhyde aromatique varie le plus souvent entre 20 et 75 % en poids, de préférence entre 25 et 60 % en poids.

Le flux liquide *(F₁₅)* est donc séparé du flux gazeux *(F₁₄)* et une fraction *(F₁₆)* du flux *(F₁₅)* est recyclée à l'alimentation de l'évaporateur et l'autre fraction *(F₁₇)* est éliminée et peut être ultérieurement valorisée.

Dans le flux liquide de sortie *(F₁₅)*, la concentration en aldéhyde aromatique varie le plus souvent entre 1 et 20 % en poids, de préférence entre 1 et 10%.

Selon une variante du procédé de l'invention, on effectue le recyclage ou la recirculation d'une fraction des lourds *(F₁₆)* afin de maintenir un film liquide sans zone sèche sur toute la hauteur des tubes ce qui permet d'améliorer le rendement de séparation et de récupération.

On recycle généralement, le flux liquide séparé comprenant les goudrons avec un taux de recyclage de 50 à 75 %.

Dans un mode de fonctionnement de l'évaporateur avec recyclage du flux *(F₁₆)*, l'alimentation *(F₁₈)* est alors composée du mélange des flux *(F₁₃)* et *(F₁₆).*

Dans le cas du recyclage d'un flux liquide *(F₁₆)*, les rapports massiques de recyclage *(F₁₆)*/*(F₁₃)* sont compris entre 1 et 10.

Les temps de séjour dans l'évaporateur sont courts : ils sont compris entre 30 secondes et 10 minutes.

L'étape de séparation de l'aldéhyde aromatique des lourds est effectuée dans l'évaporateur, par vaporisation de l'aldéhyde aromatique et des impuretés lourdes vaporisables présentes.

Le taux de vaporisation exprimé par le rapport pondéral entre le flux gazeux et le flux d'alimentation est compris entre 5 et 75 % en poids de préférence entre 5 et 50 % en poids.

Les températures d'évaporation (température moyenne dans le film) sont comprises entre 170°C et 200°C avec un gradient de température entre le haut et le bas de l'évaporateur compris entre 100°C et 20°C et une pression établie en haut d'évaporateur, comprise entre 1 et 20 mm de mercure.

La pression réduite est établie sur l'appareil par l'intermédiaire d'un circuit de vide connecté en tête d'évaporateur.

Dans cette étape de séparation par évaporation, le rendement de récupération de l'aldéhyde aromatique présent dans les lourds exprimé par le rapport pondéral de l'aldéhyde aromatique vaporisé *(F₁₄)* sur l'aldéhyde aromatique contenu dans le flux d'alimentation *(F₁₃)* est de l'ordre de 80 à 90%.

Un appareillage pour la mise en oeuvre du procédé selon l'invention est décrit.

Ce dispositif comprend une colonne de distillation de concentration des lourds et un évaporateur à film tombant ou à film ruisselant pour la séparation et la récupération de l'aldéhyde aromatique d'un mélange comprenant en outre des goudrons.

Dans un mode de réalisation, il comprend en outre au moins une colonne de distillation du solvant d'extraction.

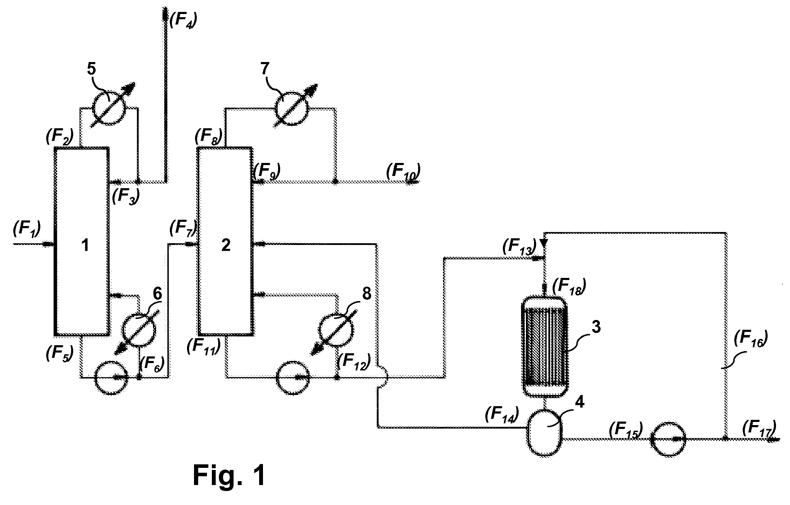

L'invention sera mieux comprise à la lecture de la description de la figure 1, annexée à la présente demande.

La figure 1 est une représentation schématique d'un dispositif préféré de mise en oeuvre du procédé selon l'invention, comprenant la colonne 1 de distillation du solvant d'extraction, la colonne de distillation 2 de concentration des lourds et l'évaporateur à film tombant ou à film ruisselant 3 couplé à cette colonne.

Sur la figure 1, le flux *(F₁)* provenant de l'étape d'extraction et comprenant les produits réactionnels dont les lourds est introduit dans la colonne de distillation 1 permettant par distillation, la séparation du solvant d'extraction de l'aldéhyde aromatique.

En tête de la colonne de distillation 1, on récupère après condensation du flux de vapeur *(F₂)* sur le condenseur 5, un flux *(F₄)* de solvant d'extraction qui pourra être recyclé à l'étape d'extraction ; une partie du flux condensé *(F₃)* est renvoyé latéralement en tête de colonne 1 et constitue le reflux de la colonne 1.

Le flux gazeux *(F₂)* est refroidi en abaissant sa température par exemple, de 15°C à 30°C, par passage dans un ou plusieurs condenseurs 5 de préférence un.

En pied de colonne de distillation 1, le flux global *(F₅)* est majoritairement re-circulé au pied de colonne en passant sur l'échangeur 6 de chauffage, le flux *(F₇)* va alimenter la colonne de distillation 2 de concentration des lourds.

En tête de la colonne 2, on récupère après condensation du flux de vapeur *(F₈)* sur le condenseur 7, un flux *(F₁₀)* de l'aldéhyde aromatique et des impuretés légères ; une partie du flux condensé *(F₉)* est renvoyé latéralement en tête de colonne 2 et constitue le reflux de la colonne 2.

Le flux *(F₁₀)* alimente le ou les dispositifs de purification finale de l'aldéhyde aromatique.

Le flux gazeux *(F₈)* est refroidi en abaissant sa température par exemple, de 15°C à 30°C, par passage dans un ou plusieurs condenseurs 7 de préférence un.

En pied de colonne de distillation 2, le flux global *(F₁₁)* est majoritairement re-circulé *(F₁₂)* au pied de colonne en passant sur l'échangeur 8 de chauffage ; le flux *(F₁₃)* va alimenter l'évaporateur à film tombant ou à film ruisselant 3.

Le flux *(F₁₃)* est introduit dans la partie supérieure de l'évaporateur 3.

Le flux sortant de l'évaporateur 3 traverse une enceinte de séparation 4 permettant de séparer un flux liquide *(F₁₅)* d'un flux gazeux *(F₁₄).*

Le flux gazeux sortant de l'enceinte de séparation 4 *(F₁₄)* est renvoyé latéralement dans la colonne de concentration 2, de préférence en bas de colonne.

Selon une variante de procédé de l'invention, il est possible de renvoyer le flux *(F₁₄)* dans la colonne, sous forme liquide par l'intermédiaire d'une pompe, après passage dans un condenseur. La pompe et le condenseur ne sont pas représentés sur la figure 1.

Une partie du flux liquide *(F₁₅)* issu de l'enceinte de séparation 4 à savoir le flux *(F₁₆)* est recyclé à l'alimentation de l'évaporateur 3 et conjointement avec le flux d'alimentation *(F₁₃)* constitue le flux global *(F₁₈)* d'alimentation de l'évaporateur 3.

L'autre partie du flux liquide *(F₁₇)* épuisé en aldéhyde aromatique comprend essentiellement les goudrons et les impuretés lourdes ; ce flux est éliminé et peut être ultérieurement valorisé.

Ainsi, conformément à une deuxième variante du procédé de l'invention, on effectue la séparation dans un évaporateur à film raclé.

On entend par évaporateur à film raclé un appareil constitué par une chambre généralement cylindrique ou cylindro-conique pourvue d'une double enveloppe externe munie d'une entrée et d'une sortie afin d'assurer la circulation d'un fluide caloporteur permettant le chauffage de la chambre.

Dans ce type d'appareil, la formation du film sur la paroi interne de la chambre est assurée par plaquage à l'aide de moyens mécaniques précisés ci-après.

La chambre est équipée en son centre, d'un rotor axial sur lequel sont montés les moyens mécaniques qui permettent la formation du film sur la paroi. Il peut s'agir de rotors équipés de pales fixes : rotors lobés à trois ou quatre pales en matériaux souples ou rigides, distribuées sur toute la hauteur du rotor ou bien des rotors équipés de pales mobiles, palettes, balais racleurs, frotteurs guidés. Dans ce cas, le rotor est constitué par une succession de palettes articulées sur pivot montées sur un arbre ou axe par l'intermédiaire de supports radiaux. D'autres rotors sont équipés de rouleaux mobiles montés sur des axes secondaires et lesdits rouleaux sont plaqués sur la paroi par centrifugation.

La vitesse de rotation du rotor qui dépend de la taille de l'appareil, est déterminée aisément par l'homme de métier. On précise à titre indicatif que la vitesse périphérique peut varier entre 0,3 et 3 m/s pour les rotors à pales mobiles ; 5 et 15 m/s pour les rotors à pales fixes.

Les différents mobiles peuvent être en matériaux divers, métalliques par exemple acier, acier allié (acier inoxydable), aluminium, ou polymériques, par exemple polytétrafluroéthylène PTFE ou des matériaux verrés (émail) ; des matériaux métalliques revêtus de matériaux polymériques.

Le mélange *(F₁₃)*, comprenant un aldéhyde aromatique, des impuretés lourdes et des goudrons, est alimenté par la partie supérieure et est centrifugé sur la paroi par l'intermédiaire d'un distributeur, par exemple un disque solidaire du rotor. Le liquide se distribue sur la paroi et forme un film liquide d'une fine épaisseur, généralement entre 0,25 et 1 mm, plaqué par les moyens mécaniques précisés ci-dessus.

Ledit mélange descend par gravité le long de la surface interne de la paroi et les fractions vaporisables (dont l'aldéhyde aromatique) sont évaporées au cours de leur cheminement le long de la paroi et la phase liquide essentiellement les lourds parvenant à la base de la chambre est récupérée.

On récupère en tête de la chambre, un flux gazeux *(F₁₄)* comprenant essentiellement l'aldéhyde aromatique et les impuretés lourdes vaporisables et en pied de la chambre, un flux liquide *(F₁₉)* comprenant essentiellement les lourds, impuretés lourdes et goudrons.

Afin de conserver le flux *(F₁₄)*, entièrement sous forme gazeuse, il est souhaitable de prévoir un dispositif de dévésiculage de gouttelettes (ou séparateur de gouttelettes) situé dans la partie supérieure de la chambre dans la zone d'évacuation du flux gazeux *(F₁₄)*.

Le flux gazeux *(F₁₄)* est à sa sortie, recyclé en amont de cette étape de séparation par évaporation, de préférence à l'étape de concentration : ce flux peut être éventuellement condensé afin d'être introduit sous forme liquide par l'intermédiaire d'une pompe.

Dans le flux gazeux *(F₁₄)*, la concentration en aldéhyde aromatique varie le plus souvent entre 25 et 75 % en poids.

Le flux liquide *(F₁₉)* est collecté à la base dans un dispositif quelconque de stockage relais, dispositif chauffé afin de maintenir le flux liquide.

Le flux liquide *(F₁₉)* séparé du flux gazeux *(F₁₄)* est fractionné : une fraction du flux *(F₂₀)* est recyclée à l'alimentation de l'évaporateur et l'autre fraction *(F₂₁)* est éliminée et peut être ultérieurement valorisée.

Dans le flux liquide de sortie *(F₁₉)*, la concentration en aldéhyde aromatique varie le plus souvent entre 1 et 20 % en poids.

Selon une variante du procédé de l'invention, on effectue le recyclage ou la recirculation d'une fraction des lourds *(F₂₀)*. Toutefois, le recyclage n'est pas impératif car le film est formé mécaniquement.

On peut recycler le flux liquide séparé comprenant les goudrons avec un taux de recyclage de 0 à 50 %.

Dans un mode de fonctionnement de l'évaporateur avec recyclage du flux *(F₂₀)*, l'alimentation *(F₁₈)* est alors composée du mélange des flux *(F₁₃)* et *(F₂₀).*

Dans le cas du recyclage d'un flux liquide *(F₂₀)*, les rapports massiques de recyclage *(F₂₀)*/*(F₁₃)* sont compris entre 0,1 et 10, de préférence 0,1 et 2.

Les temps de séjour dans l'évaporateur sont courts. Ils sont compris entre 30 secondes et 10 minutes.

L'étape de séparation de l'aldéhyde aromatique des lourds est effectuée dans l'évaporateur, par vaporisation de l'aldéhyde aromatique et des impuretés lourdes vaporisables présentes.

Le taux de vaporisation exprimé par le rapport pondéral entre le flux gazeux et le flux d'alimentation est compris entre 20 et 90 % de préférence entre 40 et 80 %.

Les températures d'évaporation (température moyenne dans le film) sont comprises entre 170°C et 200 °C avec un gradient de température entre 10°C et 20°C et une pression établie en haut d'évaporateur, comprise entre 1 et 20 mm de mercure.

La pression réduite est établie sur l'appareil par l'intermédiaire d'un circuit de vide connecté généralement en pied ou en sortie d'évaporateur.

Dans cette étape de séparation par évaporation, le rendement de récupération de l'aldéhyde aromatique exprimé par le rapport pondéral de l'aldéhyde aromatique vaporisé *(F₁₄)* sur l'aldéhyde aromatique contenu dans le flux d'alimentation *(F₁₃)* varie le plus souvent entre 85 et 99 %, de préférence entre 90 et 99 %.

Un appareillage pour la mise en oeuvre du procédé selon l'invention est décrit.

Ce dispositif comprend une colonne de distillation de concentration des lourds et un évaporateur à film raclé pour la séparation et la récupération de l'aldéhyde aromatique d'un mélange comprenant en outre des goudrons.

Dans un mode de réalisation, il comprend en outre au moins une colonne de distillation du solvant d'extraction.

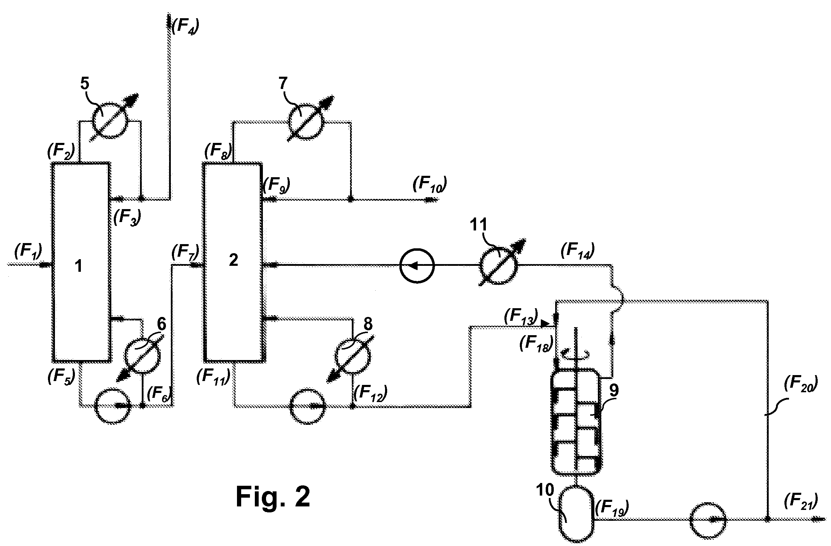

La figure 2 est une représentation schématique d'un dispositif préféré de mise en oeuvre du procédé selon l'invention, comprenant la colonne 1 de distillation du solvant d'extraction, la colonne de distillation 2 de concentration des lourds et l'évaporateur à film raclé 9 couplé à cette colonne.

Sur la figure 2, le flux *(F₁₃)* obtenu comme mentionné pour la figure 1, est introduit dans la partie supérieure de l'évaporateur 9.

Le flux liquide *(F₁₉)* est récupéré en pied de l'évaporateur 9.

Le flux gazeux *(F₁₄)* est récupéré en tête de l'évaporateur 9 et renvoyé latéralement dans la colonne 2 de concentration, de préférence en bas de colonne.

Selon une variante préférée de procédé de l'invention, il est possible de renvoyer le flux *(F₁₄)* dans la colonne 2, sous forme liquide par l'intermédiaire d'une pompe, après passage dans un condenseur 11.

Une partie du flux liquide *(F₁₉)* récupéré en pied d'évaporateur dans le dispositif 10 est recyclé à l'alimentation de l'évaporateur 9 et conjointement avec le flux d'alimentation *(F₁₃)* constitue le flux global *(F₁₈)* d'alimentation de l'évaporateur 9.

L'autre partie du flux liquide *(F₂₁)* épuisé en aldéhyde aromatique comprend essentiellement les goudrons et les impuretés lourdes ; ce flux est éliminé et peut être ultérieurement valorisé.

Ainsi, conformément à une troisième variante du procédé de l'invention, on effectue la séparation dans un évaporateur à court-trajet à condenseur interne.

On entend par évaporateur à court-trajet à condenseur interne, un appareil constitué par une chambre généralement cylindrique pourvue d'une double enveloppe externe munie d'une entrée et d'une sortie afin d'assurer la circulation d'un fluide caloporteur assurant le chauffage de la chambre.

Dans ce type d'appareil, la formation du film sur la paroi interne de la chambre est assurée par plaquage à l'aide de moyens mécaniques précisés ci-après.

La chambre est équipée d'un rotor axial dans la partie supérieure de la chambre et en dessous, d'un condenseur interne central.

Sur un dispositif solidaire du rotor central, sont installés des axes secondaires, tiges verticales sur lesquelles sont montés des rouleaux coulissants.

Les différents rouleaux peuvent être en matériaux divers, métalliques par exemple acier, acier allié (acier inoxydable), aluminium, ou polymériques, par exemple polytétrafluroéthylène PTFE ou matériaux métalliques revêtus de matériaux polymériques.

La formation du film sur la paroi est assurée par la centrifugation et le plaquage des rouleaux sur la paroi, par rotation du rotor.

La vitesse de rotation du rotor qui dépend de la taille de l'appareil, est déterminée aisément par l'homme de métier. On précise à titre indicatif que la vitesse périphérique peut varier entre 0,3 et 3 m/s.

Le condenseur placé au centre de la chambre permet de condenser les vapeurs au sein même du dispositif d'évaporation. Il peut s'agir d'un échangeur tubulaire, par exemple serpentins ou faisceau de tubes.

Le mélange *(F₁₃)*, comprenant un aldéhyde aromatique, des impuretés lourdes et des goudrons, est alimenté par la partie supérieure et est centrifugé sur la paroi par l'intermédiaire d'un distributeur, par exemple un disque solidaire du rotor. Le liquide se distribue sur la paroi et forme un film liquide d'une fine épaisseur, généralement entre 0,25 et 1 mm, plaqué par les moyens mécaniques précisés ci-dessus.

Ledit mélange s'écoule par gravité le long de la surface interne de la paroi et les fractions vaporisables (dont l'aldéhyde aromatique) sont évaporées et immédiatement condensées à l'aide du condenseur central et la fraction condensée *(F₁₄)* s'écoule gravitairement sur les parois du condenseur interne et les vapeurs condensées sont collectées à la base de l'appareil dans un collecteur interne.

On récupère une phase liquide *(F₁₄)* dans ledit collecteur qui comprend essentiellement l'aldéhyde aromatique et les impuretés lourdes vaporisables et en pied de la chambre, un flux liquide *(F₁₅)* comprenant essentiellement les lourds, impuretés lourdes et goudrons.

Selon une variante préférée du procédé de l'invention, le flux liquide *(F₁₄)* est à sa sortie, recyclé en amont de cette étape de séparation par évaporation, plus particulièrement à l'étape de concentration : ce flux est introduit par l'intermédiaire d'une pompe.

Dans le flux liquide *(F₁₄)*, la concentration en aldéhyde aromatique varie le plus souvent entre 25 et 75 % en poids.

Le flux liquide non vaporisé *(F₁₅)* est collecté à la base dans un dispositif quelconque de stockage relais, dispositif chauffé afin de maintenir le flux liquide.

Le flux liquide *(F₁₅)* peut être ultérieurement valorisé.

Dans le flux liquide de sortie *(F₁₅)*, la concentration en aldéhyde aromatique varie le plus souvent entre 1 et 15 % en poids.

Les temps de séjour dans l'évaporateur sont courts. Ils sont compris entre 30 secondes et 10 minutes.

L'étape de séparation de l'aldéhyde aromatique des lourds est effectuée dans l'évaporateur, par vaporisation de l'aldéhyde aromatique et des impuretés lourdes vaporisables présentes.

Le taux de vaporisation exprimé par le rapport pondéral entre le flux gazeux et le flux d'alimentation est compris entre 10 et 95 % de préférence entre 15 et 90 %.

Les températures d'évaporation (température moyenne dans le film) sont comprises entre 170°C et 200°C avec un gradient de température entre le haut et le bas de l'évaporateur compris entre 10°C et 20°C et une pression établie en haut d'évaporateur, comprise entre 0,01 et 20 mm de mercure, de préférence entre 0,1 à 10 mm de mercure.

La pression réduite est établie sur l'appareil par l'intermédiaire d'un circuit de vide connecté généralement en pied ou en sortie d'évaporateur.

Dans cette étape de séparation par évaporation, le rendement de récupération de l'aldéhyde aromatique exprimé par le rapport pondéral de l'aldéhyrde aromatique vaporisé *(F₁₄)* sur l'aldéhyde aromatique contenu dans le flux d'alimentation *(F₁₃)* varie le plus souvent entre 85 et 99 %, de préférence entre 90 et 99 %.

Un appareillage pour la mise en oeuvre du procédé selon l'invention est décrit.

Ce dispositif comprend une colonne de distillation de concentration des lourds et un évaporateur à court-trajet à condenseur interne pour la séparation et la récupération de l'aldéhyde aromatique d'un mélange comprenant en outre des goudrons.

Dans un mode de réalisation, il comprend en outre au moins une colonne de distillation du solvant d'extraction.

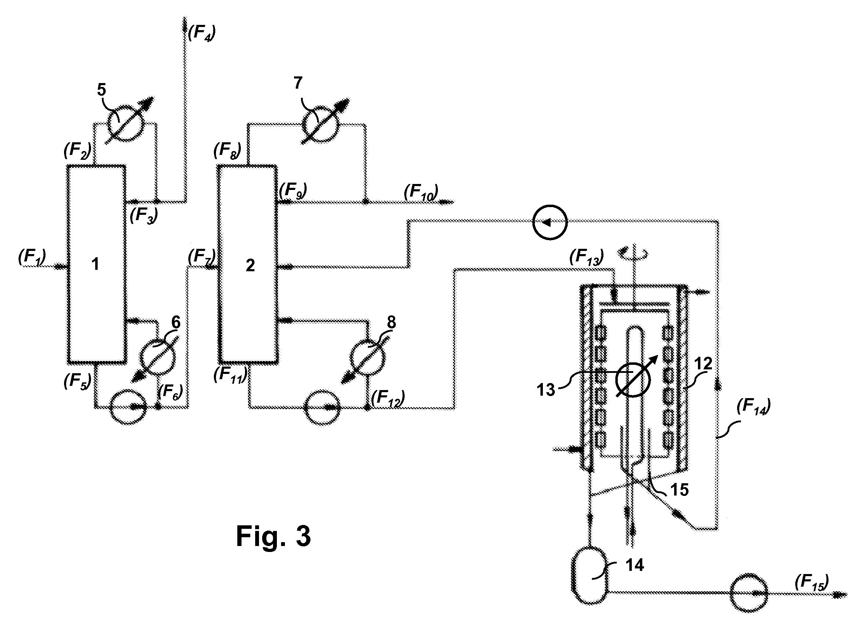

La figure 3 est une représentation schématique d'un dispositif préféré de mise en oeuvre du procédé selon l'invention, comprenant la colonne 1 de distillation du solvant d'extraction, la colonne de distillation 2 de concentration des lourds 2 et l'évaporateur à court-trajet 12 à condenseur interne 13, couplé à cette colonne.

Sur la figure 3, le flux *(F₁₃)* obtenu comme mentionné pour la figure 1, est introduit dans la partie supérieure de l'évaporateur 12.

Le flux liquide *(F₁₅)* est récupéré en pied de l'évaporateur 12.

Le flux gazeux *(F₁₄)* condensé à l'intérieur de l'évaporateur sur le condenseur 13 est récupéré en pied de l'évaporateur 12 dans le collecteur de condensats 15 et renvoyé latéralement dans la colonne de concentration 2, de préférence en bas de colonne. Le flux *(F₁₄)* qui est liquide est renvoyé par l'intermédiaire d'une pompe.

Le flux liquide *(F₁₅) est* collecté dans un dispositif de stockage 14.

Il comprend essentiellement les goudrons et les impuretés lourdes qui sont évacués et peuvent être ultérieurement valorisés.

Ainsi, conformément à une quatrième variante du procédé de l'invention, on effectue la séparation dans un évaporateur raclé horizontal.

On entend par évaporateur à film raclé horizontal, un appareil constitué par une chambre généralement cylindrique placée horizontalement pourvue d'une double enveloppe externe munie d'une entrée et d'une sortie afin d'assurer la circulation d'un fluide caloporteur assurant le chauffage de la chambre.

Dans ce type d'appareil, la formation du film sur la paroi interne de la chambre est assurée par plaquage à l'aide de moyens mécaniques précisés ci-après.

La chambre est équipée en son centre, d'un rotor axial horizontal, éventuellement chauffé par circulation interne d'un caloporteur, sur lequel sont montés les moyens mécaniques qui permettent la formation du film sur la paroi. Il peut s'agir de rotors équipés par une succession de palettes fixes montées à la périphérie du rotor. La disposition des palettes sur le rotor est telle qu'elle provoque l'avancement du film vers l'extrémité de sortie de la chambre, à l'opposé de l'alimentation ; profil de déplacement d'une vis sans fin. Il est possible selon le dispositif de prévoir des systèmes de contre-pales profilées sur la paroi permettant grâce à une interaction avec les palettes, le nettoyage automatique des surfaces internes de l'évaporateur et du rotor.

Il est également possible d'avoir dans une chambre deux rotors en parallèle qui tournent dans le même sens ou en sens inverse.

Les différents mobiles sont généralement en matériaux métalliques, le plus souvent un acier ou un acier allier (acier inoxydable).

Le mélange *(F₁₃)*, comprenant l'aldéhyde aromatique, les impuretés lourdes et les goudrons, est alimenté à une extrémité de la chambre de l'évaporateur et sur le dessus de celle-ci. Le liquide se distribue sur la paroi et l'épaisseur du film liquide est ajustée par le jeu mécanique entre les pales fixes du rotor et la paroi interne de la chambre équipée de contre-pales.

L'épaisseur du film varie généralement entre 1 et 3 mm. L'appareil permet un écoulement préférentiellement piston du liquide en direction de la sortie.

Ledit mélange avance transversalement dans la chambre et les fractions vaporisables *(F₁₄)* comprenant essentiellement l'aldéhyde aromatique et les impuretés lourdes vaporisables sont évaporées et collectées par une tubulure de sortie située sur le dessus de la chambre et sont ensuite envoyées dans un condenseur.

La tubulure d'évacuation des fractions vaporisables peut être équipée d'un filtre maintenu à une température supérieure à celle des vapeurs.

La phase liquide *(F₁₅)* comprenant essentiellement les lourds, impuretés lourdes et goudrons est récupérée à l'extrémité opposée de l'alimentation dans un dispositif de stockage relais.

Le flux gazeux *(F₁₄)* est à sa sortie, recyclé en amont de cette étape de séparation par évaporation.

Selon une variante préférée du procédé de l'invention, il est recyclé à l'étape de concentration : ce flux est condensé afin d'être introduit sous forme liquide par l'intermédiaire d'une pompe.

Dans le flux gazeux *(F₁₄)*, la concentration en aldéhyde aromatique varie le plus souvent entre 25 et 75 % en poids.

Le flux liquide *(F₁₅)* est collecté à la base dans un dispositif quelconque de stockage relais, dispositif chauffé afin de maintenir le flux liquide.

Dans le flux liquide de sortie *(F₁₅)*, la concentration en aldéhyde aromatique varie le plus souvent entre 1 et 15 % en poids ; ce flux est éliminé et peut être ultérieurement valorisé.

Les temps de séjour dans l'évaporateur sont compris entre 30 secondes et 10 minutes.

L'étape de séparation de l'aldéhyde aromatique des lourds est effectuée dans l'évaporateur, par vaporisation de l'aldéhyde aromatique et des impuretés lourdes vaporisables présentes.

Le taux de vaporisation exprimé par le rapport pondéral entre le flux gazeux et le flux d'alimentation est compris entre 10 et 90 %, de préférence entre 15 et 90 %.

Les températures d'évaporation (température moyenne dans le film) sont comprises entre 170°C et 200°C avec un gradient de température entre l'entrée et la sortie de l'évaporateur compris entre 10°C et 20°C et une pression établie en haut d'évaporateur, comprise entre 2 et 20 mm de mercure.

La pression réduite est établie sur l'appareil par l'intermédiaire d'un circuit de vide connecté généralement en haut d'évaporateur.

Dans cette étape de séparation par évaporation, le rendement de récupération de l'aldéhyde aromatique exprimé par le rapport pondéral de l'aldéhyde aromatique vaporisé *(F₁₄)* sur l'aldéhyde aromatique contenu dans le flux d'alimentation *(F₁₃)* varie le plus souvent entre 90 et 99 %, de préférence entre 95 et 99 %.

Un appareillage pour la mise en oeuvre du procédé selon l'invention est décrit.

Ce dispositif comprend une colonne de distillation de concentration des lourds et un évaporateur à film raclé horizontal pour la séparation et la récupération de l'aldéhyde aromatique d'un mélange comprenant en outre des goudrons.

Dans un mode de réalisation préféré, il comprend en outre une colonne de distillation du solvant d'extraction.

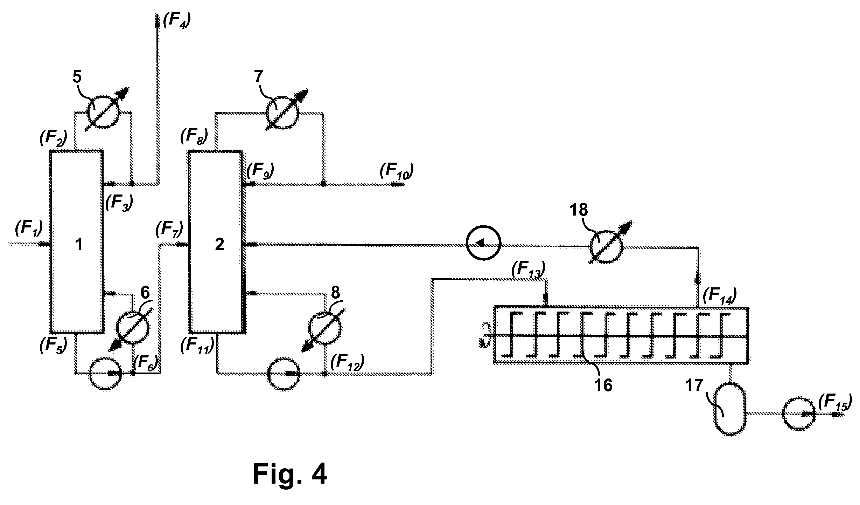

La figure 4 est une représentation schématique d'un dispositif préféré de mise en oeuvre du procédé selon l'invention, comprenant la colonne 1 de distillation du solvant d'extraction, la colonne de distillation 2 de concentration des lourds et l'évaporateur à film raclé horizontal 16 couplé à cette colonne.

Sur la figure 4, le flux *(F₁₃)* obtenu comme mentionné pour la figure 1, est introduit en partie haute de l'évaporateur 16 et à une extrémité de celui-ci.

Le flux liquide *(F₁₅)* est récupéré à l'autre extrémité de l'évaporateur 16.

Le flux gazeux *(F₁₄)* est récupéré sur le dessus de l'évaporateur 16 et renvoyé latéralement dans la colonne de concentration 2, de préférence en bas de colonne, après condensation sur le condenseur 18 et transfert par pompe par exemple volumétrique.

Le flux liquide *(F₁₅)* qui comprend essentiellement les goudrons et les impuretés lourdes est collecté dans un dispositif de stockage relais 17 ; ce flux est éliminé et peut être ultérieurement valorisé.

Comme décrit précédemment, la séparation selon l'invention peut être effectuée dans un évaporateur à film tombant ou ruisselant, à film raclé, à court trajet ou à film raclé horizontal. L'invention inclut le cas où plusieurs évaporateurs peuvent être montés en série. On peut citer notamment l'utilisation d'un évaporateur horizontal couplé à la sortie d'un autre type d'évaporateurs tels que précités.

Conformément au procédé de l'invention, la séparation de l'aldéhyde aromatique s'inscrit dans un procédé en continu avec introduction des flux et récupération des flux en continu. Toutefois, l'invention n'exclut pas un mode de réalisation en discontinu.

La présente invention permet l'élimination des lourds et un recyclage en continu des flux séparés dans une installation industrielle.

Le procédé de l'invention permet la récupération de l'aldéhyde aromatique présent dans les lourds avec des temps de séjour suffisamment courts et des conditions de température et de pression qui permettent de respecter l'intégrité du produit à séparer, tout en étant simple à mettre en oeuvre.

On donne ci-après des exemples de réalisation de l'invention obtenus sur une installation pilote fonctionnant en mode continu.

Les exemples sont donnés à titre illustratif et sans caractère limitatif.

Dans les exemples, les analyses des flux (dont la vanilline) sont effectuées par chromatographie en phase liquide, haute performance.

Comme mentionné précédemment dans la description, tout l'appareillage est maintenu sous atmosphère d'azote.

### Exemple 1

Dans cet exemple, on illustre le procédé qui permet de récupérer la vanilline présente dans des goudrons, en utilisant un évaporateur à film tombant tel qu'illustré sur la figure 1 destiné à traiter les goudrons obtenus après l'extraction liquide/liquide d'un mélange réactionnel issu de la synthèse de la vanilline (VA).

Le flux *(F₁)* qui alimente la colonne 1 a une teneur en vanilline de 0,4000 kg/kg, une teneur en impuretés légères de 0,0144 kg/kg et une teneur en impuretés lourdes de 0,0084 kg/kg.

Le flux *(F₁)* est introduit dans la colonne 1 avec un débit d'alimentation de 211,48 kg/h.

En tête de colonne 1, on récupère après condensation un flux liquide *(F₄)* à raison de 122,07 kg/h et qui ne contient plus de vanilline.

En pied de colonne 1, on soutire un flux liquide *(F₅)* à raison de 600 kg/h et comprenant 0,9461 kg de vanilline par kg de flux *(F₅).*

Le flux *(F₇)* est ensuite introduit dans la colonne de distillation 2 avec un débit d'alimentation de 89,41 kg/h. Sa teneur en vanilline est de 0,9461 kg/kg.

La colonne 2 utilisée présente les caractéristiques et les conditions de fonctionnement suivantes :

**Tableau (I)**

| **Colonne 2** | |
|---|---|
| Diamètre (mm) | 200 |
| Hauteur (mm) | 2500 |
| NET (nombre étages théoriques) total bouilleur inclus | 11 |
| NET (nombre étages théoriques) au dessous de l'alimentation | 9 |
| Pression en tête de colonne 2 (mm Hg) | 8 |
| Pression en pied de colonne 2 (mm Hg) | 16 |
| Température tête de colonne 2 (°C) | 145 |
| Température pied de colonne 2 (°C) | 181 |
| Facteur de charge (kg/m³)^{0,5} ×m.s⁻¹ | 4,3 |
| Volatilité relative moyenne (VA/lourds) | 8,0 |
| Reflux de tête de colonne 2 : *(F₉)* après condenseur 7 (kg/h) | 21,9 |
| Recyclage de pied de colonne 2 : (*F₁₂)* sur échangeur 8 (kg/h) | 550 |
| Charge thermique au bouilleur (kcal/h) | 13050 |

En tête de colonne 2, on récupère un flux gazeux *(F₈)* qui après condensation devient le flux *(F₁₀)* à raison de 87,5 kg/h et qui contient de la vanilline à une teneur de 0,9645 kg/kg.

En pied de colonne 2, on soutire un flux liquide *(F₁₁)* à raison de 554,2 kg/h et comprenant 0,2965 kg de vanilline par kg de flux *(F₁₁)*.

Une fraction *(F₁₃)* du flux *(F₁₁)* alimente l'évaporateur à film tombant 3, l'autre fraction *(F₁₂)* étant réintroduite latéralement dans la colonne de distillation 2.

Les caractéristiques et les conditions de fonctionnement de l'évaporateur 3 sont rassemblées dans le tableau (II) :

**Tableau (II)**

| **Evaporateur à film tombant 3** | |
|---|---|
| Nombre de tubes | 10 |
| Diamètre d'un tube (mm) | 10 |
| Epaisseur tube (mm) | 2 |
| Hauteur (mm) | 800 |
| Surface échange totale (m²) | 0,251 |
| Pression évaporateur (mm Hg) | 14 |
| Température caloporteur (°C) | 195 |
| Température moyenne de film (°C) | 185 |
| Viscosité moyenne de l'alimentation (Cpₒ) | 150 |
| Coef. de transfert de chaleur global (kcal/hm²°C) | 180 |
| Charge thermique de l'évaporateur à film tombant (kcal/h) | 454 |
| Volatilité relative moyenneVA/lourds (mmHg/mmHg) | 5,6 |
| | |
| Alimentation depuis colonne 2 : (*F₁₃)* (kg/h) | 4,198 |
| Teneur en VA dans (*F₁₃)* (kg/kg) | 0,297 |
| Alimentation totale (*F₁₈)* (kg/h) | 29,50 |
| Teneur en VA dans (*F₁₈)* (kg/kg) | 0,1317 |
| Vaporisation et recyclage à la colonne 2 : (*F₁₄)* (kg/h) | 2,288 |
| Teneur en VA dans (*F₁₄)* (kg/kg) | 0,456 |
| Liquide en pied d'évaporateur : (*F₁₅)* (kg/h) | 27,23 |
| Teneur en VA dans (*F₁₅)* (kg/kg) | 0,104 |
| Recyclage à l'alimentation du film tombant : (*F₁₆)* (kg/h) | 25,32 |
| Teneur en VA dans (*F₁₆)* (kg/kg) | 0,104 |
| Goudrons extraits : (*F₁₇)* (kg/h) | 1,909 |
| Teneur en VA dans (*F₁₇)* (kg/kg) | 0,104 |
| Pertes VA dans (*F₁₇)* (kg/h) | 0,199 |
| Taux de vaporisation de l'évaporateur à film tombant (% masse) | 7,7 |
| **Rendement de récupération évaporateur à film tombant en VA (% masse)** | **83,8** |

### Exemple 2

Dans cet exemple, on illustre le procédé qui permet de récupérer la vanilline présente dans des goudrons, en utilisant un évaporateur à film tombant tel qu'illustré sur la figure 1 destiné à traiter les goudrons obtenus après l'extraction liquide/liquide d'un mélange réactionnel issu de la synthèse de la vanilline (VA).

Le flux *(F₁)* qui alimente la colonne 1 a une teneur en vanilline de 0,6000 kg/kg, une teneur en impuretés légères de 0,0215 kg/kg et une teneur en impuretés lourdes de 0,0127 kg/kg.

Le flux *(F₁)* est introduit dans la colonne 1 avec un débit d'alimentation de 78,84 kg/h.

En tête de colonne 1, on récupère après condensation un flux liquide *(F₄)* à raison de 28,80 kg/h et qui ne contient plus de vanilline.

En pied de colonne 1, on soutire un flux liquide *(F₅)* à raison de 500 kg/h et comprenant 0,9461 kg de vanilline par kg de flux *(F₅).*

Le flux *(F₇)* est ensuite introduit dans la colonne de distillation 2 avec un débit d'alimentation de 50,00 kg/h. Sa teneur en vanilline est de 0,9461 kg/kg.

La colonne 2 utilisée présente les caractéristiques et les conditions de fonctionnement suivantes :

**Tableau (III)**

| **Colonne 2** | |
|---|---|
| Diamètre (mm) | 200 |
| Hauteur (mm) | 2500 |
| NET (nombre étages théoriques) total bouilleur inclus | 11 |
| NET (nombre étages théoriques) au dessous de l'alimentation | 9 |
| Pression en tête de colonne 2 (mm Hg) | 2 |
| Pression en pied de colonne 2 (mm Hg) | 7 |
| Température tête de colonne 2 (°C) | 121 |
| Température pied de colonne 2 (°C) | 171 |
| Facteur de charge (kg/m³)^{0,5} ×m.s⁻¹ | 3,8 |
| Volatilité relative moyenne (VA/lourds) | 8,0 |
| Reflux de tête de colonne 2 : *(F₉)* après condenseur 7 (kg/h) | 12,3 |
| Recyclage de pied de colonne 2 : *(F₁₂)* sur échangeur 8 (kg/h) | 550 |
| Charge thermique au bouilleur (kcal/h) | 7060 |

En tête de colonne 2, on récupère un flux gazeux *(F₈)* qui après condensation devient le flux *(F₁₀)* à raison de 49,0 kg/h et qui contient de la vanilline à une teneur de 0,9646 kg/kg.

En pied de colonne 2, on soutire un flux liquide *(F₁₁)* à raison de 551,8 kg/h et comprenant 0,1260 kg de vanilline par kg de flux *(F₁₁)*.

Une fraction *(F₁₃)* du flux *(F₁₁)* alimente l'évaporateur à film tombant 3, l'autre fraction *(F₁₂)* étant réintroduite latéralement dans la colonne de distillation 2.

Les caractéristiques et les conditions de fonctionnement de l'évaporateur 3 sont rassemblées dans le tableau (IV) :

**Tableau (IV)**

| **Evaporateur à film tombant 3** | |
|---|---|
| Nombre de tubes | 10 |
| Diamètre d'un tube (mm) | 10 |
| Epaisseur tube (mm) | 2 |
| Hauteur (mm) | 800 |
| Surface échange totale (m²) | 0,251 |
| Pression évaporateur (mm Hg) | 6,6 |
| Température caloporteur (°C) | 196,5 |
| Température moyenne de film (°C) | 194 |
| Viscosité moyenne de l'alimentation (Cpₒ) | 120 |
| Coef. de transfert de chaleur global (kcal/hm²°C) | 173 |
| Charge thermique de l'évaporateur à film tombant (kcal/h) | 89 |
| Volatilité relative moyenneVA/lourds (mmHg/mmHg) | 8,0 |
| | |
| Alimentation depuis colonne 2 : *(F₁₃)* (kg/h) | 1,826 |
| Teneur en VA dans (*F₁₃)* (kg/kg) | 0,126 |
| Alimentation totale (*F₁₈)* (kg/h) | 13,33 |
| Teneur en VA dans (*F₁₈)* (kg/kg) | 0,0495 |
| Vaporisation et recyclage à la colonne 2 : (*F₁₄)* (kg/h) | 0,826 |
| Teneur en VA dans (*F₁₄)* (kg/kg) | 0,234 |
| Liquide en pied d'évaporateur : (*F₁₅)* (kg/h) | 12,48 |
| Teneur en VA dans (*F₁₅)* (kg/kg) | 0,037 |
| Recyclage à l'alimentation du film tombant : (*F₁₆)* (kg/h) | 11,52 |
| Teneur en VA dans (*F₁₆)* (kg/kg) | 0,037 |
| Goudrons extraits : (*F₁₇)* (kg/h) | 0,960 |
| Teneur en VA dans (*F₁₇)* (kg/kg) | 0,037 |
| Pertes VA dans (*F₁₇)* (kg/h) | 0,037 |
| Taux de vaporisation de l'évaporateur à film tombant (% masse) | 6,5 |
| **Rendement de récupération évaporateur à film tombant en VA (% masse)** | **84,3** |

### Exemple 3

Dans cet exemple, on met en oeuvre un évaporateur à film raclé 9 tel qu'illustré sur la figure 2.

Le flux *(F₁)* qui alimente la colonne 1 a une teneur en vanilline de 0,6000 kg/kg, une teneur en impuretés légères de 0,0215 kg/kg et une teneur en impuretés lourdes de 0,0127 kg/kg.

Le flux *(F₁)* est introduit dans la colonne 1 avec un débit d'alimentation de 78,84 kg/h.

En tête de colonne 1, on récupère après condensation un flux liquide *(F₄)* à raison de 28,80 kg/h et qui ne contient plus de vanilline.

En pied de colonne 1, on soutire un flux liquide *(F₅)* à raison de 550 kg/h et comprenant 0,9461 kg de vanilline par kg de flux *(F₅).*

Le flux *(F₇)* est ensuite introduit dans la colonne de distillation 2 avec un débit d'alimentation de 50,00 kg/h. Sa teneur en vanilline est de 0,9461 kg/kg.

La colonne 2 utilisée présente les caractéristiques et les conditions de fonctionnement suivantes :

**Tableau (V)**

| **Colonne 2** | |
|---|---|
| Diamètre (mm) | 200 |
| Hauteur (mm) | 2500 |
| NET (nombre étages théoriques) total bouilleur inclus | 11 |
| NET (nombre étages théoriques) au dessous de l'alimentation | 9 |
| Pression en tête de colonne 2 (mm Hg) | 2 |
| Pression en pied de colonne 2 (mm Hg) | 9,5 |
| Température tête de colonne 2 (°C) | 121 |
| Température pied de colonne 2 (°C) | 160 |
| Facteur de charge (kg/m³)^{0,5} ×m.s⁻¹ | 3,9 |
| Volatilité relative moyenne (VA/lourds) | 8,1 |
| Reflux de tête de colonne 2 : *(F₉)* après condenseur 7 (kg/h) | 14,6 |
| Recyclage de pied de colonne 2 : *(F₁₂)* sur échangeur 8 (kg/h) | 600 |
| Charge thermique au bouilleur (kcal/h) | 6985 |

En tête de colonne 2, on récupère un flux gazeux *(F₈)* qui après condensation devient le flux *(F₁₀)* à raison de 48,8 kg/h et qui contient de la vanilline à une teneur de 0,9646 kg/kg.

En pied de colonne 2, on soutire un flux liquide *(F₁₁)* à raison de 604,9 kg/h et comprenant 0,531 kg de vanilline par kg de flux *(F₁₁)*.

Une fraction *(F₁₃)* du flux *(F₁₁)* alimente l'évaporateur à film raclé 9, l'autre fraction *(F₁₂)* étant réintroduit latéralement dans la colonne de distillation 2.

Les caractéristiques et les conditions de fonctionnement de l'évaporateur 9 sont rassemblées dans le tableau (VI) :

**Tableau (VI)**

| **Evaporateur à film raclé 9** | |
|---|---|
| Nombre de tubes | 1 |
| Diamètre du tube (mm) | 100 |
| Epaisseur de tube (mm) | 4 |
| Hauteur (mm) | 700 |
| Surface échange (m²) | 0,22 |
| Nature des pales mobiles de raclage (aluminium) | rouleaux |
| Vitesse de rotation du rotor (tours/mn) | 300 |
| Pression évaporateur (mm Hg) | 2 |
| Température caloporteur (°C) | 207 |
| Température moyenne de film (°C) | 184 |
| Viscosité moyenne de l'alimentation (Cpₒ) | 120 |
| Coef. de transfert de chaleur global (kcal/hm²°C) | 82 |
| Charge thermique de l'évaporateur à film raclé (kcal/h) | 398 |
| Volatilité relative moyenne (VA/lourds) | 8,4 |
| | |
| Alimentation depuis colonne 2 : *(F₁₃)* (kg/h) | 4,877 |
| Teneur en VA dans *(F₁₃)* (kg/kg) | 0,5305 |
| Alimentation totale *(F₁₈)* (kg/h) | 4,877 |
| Teneur en VA dans *(F₁₈)* (kg/kg) | 0,5305 |
| Vaporisation et recyclage à la colonne 2 : *(F₁₄)* (kg/h) | 3,678 |
| Teneur en VA dans *(F₁₄)* (kg/kg) | 0,0641 |
| Liquide en pied d'évaporateur : *(F₁₉)* (kg/h) | 1,199 |
| Teneur en VA dans *(F₁₉)* (kg/kg) | 0,192 |
| Recyclage à l'alimentation du film raclé : *(F₂₀)* (kg/h) | 0 |
| Goudrons extraits : *(F₂₁)* (kg/h) | 1,199 |
| Teneur en VA dans *(F₂₁)* (kg/kg) | 0,192 |
| Pertes VA dans *(F₂₁)* (kg/h) | 0,229 |
| Taux de vaporisation de l'évaporateur à film raclé (% masse) | 75,4 |
| **Rendement de récupération évaporateur à film raclé en VA (% masse)** | **91,1** |

### Exemple 4

Dans cet exemple, on met en oeuvre un évaporateur court-trajet (à condenseur interne) 12 tel qu'illustré sur la figure 3.

Le flux *(F₁)* qui alimente la colonne 1 a une teneur en vanilline de 0,6000 kg/kg, une teneur en impuretés légères de 0,0215 kg/kg et une teneur en impuretés lourdes de 0,0127 kg/kg.

Le flux *(F₁)* est introduit dans la colonne 1 avec un débit d'alimentation de 78,84 kg/h.

En tête de colonne 1, on récupère après condensation un flux liquide *(F₄)* à raison de 28,80 kg/h et qui ne contient plus de vanilline.

En pied de colonne 1, on soutire un flux liquide *(F₅)* à raison de 550 kg/h et comprenant 0,9461 kg de vanilline par kg de flux *(F₅)*.

Le flux *(F₇)* est ensuite introduit dans la colonne de distillation 2 avec un débit d'alimentation de 50,00 kg/h. Sa teneur en vanilline est de 0,946 kg/kg.

La colonne 2 utilisée présente les caractéristiques et les conditions de fonctionnement suivantes :

**Tableau (VII)**

| **Colonne 2** | |
|---|---|
| Diamètre (mm) | 200 |
| Hauteur (mm) | 2500 |
| NET (nombre étages théoriques) total bouilleur inclus | 11 |
| NET (nombre étages théoriques) au dessous de l'alimentation | 9 |
| Pression en tête de colonne 2 (mm Hg) | 2 |
| Pression en pied de colonne 2 (mm Hg) | 9,5 |
| Température tête de colonne 2 (°C) | 121 |
| Température pied de colonne 2 (°C) | 174 |
| Facteur de charge (kg/m³)^{0,5} ×m.s⁻¹ | 4,3 |
| Volatilité relative moyenne (VA/lourds) | 8,1 |
| Reflux de tête de colonne 2 : *(F₉)* après condenseur 7 (kg/h) | 14,7 |
| Recyclage de pied de colonne 2 : *(F₁₂)* sur échangeur 8 (kg/h) | 500 |
| Charge thermique au bouilleur (kcal/h) | 7500 |

En tête de colonne 2, on récupère un flux gazeux *(F₈)* qui après condensation devient le flux *(F₁₀)* à raison de 48,99 kg/h et qui contient de la vanilline à une teneur de 0,9648 kg/kg.

En pied de colonne 2, on soutire un flux liquide *(F₁₁)* à raison de 503,3 kg/h et comprenant 0,1898 kg de vanilline par kg de flux *(F₁₁)*.

Une fraction *(F₁₃)* du flux *(F₁₁)* alimente l'évaporateur à court trajet à condenseur interne 12, l'autre fraction *(F₁₂)* étant réintroduit latéralement dans la colonne de distillation 2.

Les caractéristiques et les conditions de fonctionnement de l'évaporateur 12 sont rassemblées dans le tableau (VIII) :

**Tableau (VIII)**

| **Evaporateur à court trajet à condenseur interne 12** | |
|---|---|
| Nombre de tubes | 1 |
| Diamètre du tube (mm) | 90 |
| Epaisseur de tube (mm) | 4 |
| Hauteur (mm) | 170 |
| Surface échange (m²) | 0,048 |
| Nature des pales mobiles de raclage (teflon) | rouleaux |
| Vitesse de rotation du rotor (tours/mn) | 350 |
| Pression évaporateur (mm Hg) | 4 |
| Température caloporteur (°C) | 222 |
| Température moyenne de film (°C) | 192 |
| Viscosité moyenne de l'alimentation (Cpₒ) | 120 |
| Coef. de transfert de chaleur global (kcal/hm²°C) | 91 |
| Charge thermique de l'évaporateur à film raclé (kcal/h) | 118 |
| Volatilité relative moyenne (VA/lourds) | 7,9 |
| | |
| Alimentation depuis colonne 2 : *(F₁₃)* (kg/h) | 3,313 |
| Teneur en VA dans *(F₁₃)* (kg/kg) | 0,1897 |
| Vaporisation et recyclage à la colonne 2 : *(F₁₄)* (kg/h) | 2,304 |
| Teneur en VA dans *(F₁₄)* (kg/kg) | 0,2552 |
| Liquide en pied d'évaporateur : *(F₁₅)* (kg/h) | 1,009 |
| Teneur en VA dans *(F₁₅)* (kg/kg) | 0,0404 |
| Goudrons extraits : *(F₁₅)* (kg/h) | 1,009 |
| Teneur en VA dans *(F₁₅)* (kg/kg) | 0,0404 |
| Pertes VA dans *(F₁₅)* (kg/h) | 0,0404 |
| Taux de vaporisation dans l'évaporateur à court trajet à condenseur interne (% masse) | 69 |
| **Rendement de récupération évaporateur à court trajet à condenseur interne en VA (% masse)** | **93,56** |

### Exemple 5

Dans cet exemple, on met en oeuvre un évaporateur à film raclé horizontal 16 (rotor à pale fixes autonettoyantes) tel qu'illustré sur la figure 4.

Le flux *(F₁)* qui alimente la colonne 1 a une teneur en vanilline de 0,6000 kg/kg, une teneur en impuretés légères de 0,0215 kg/kg et une teneur en impuretés lourdes de 0,0127 kg/kg.

Le flux *(F₁)* est introduit dans la colonne 1 avec un débit d'alimentation de 78,84 kg/h.

En tête de colonne 1, on récupère après condensation un flux liquide *(F₄)* à raison de 28,80 kg/h et qui ne contient plus de vanilline.

En pied de colonne 1, on soutire un flux liquide *(F₅)* à raison de 500 kg/h et comprenant 0,9461 kg de vanilline par kg de flux *(F₅)*.

Le flux *(F₇)* est ensuite introduit dans la colonne de distillation 2 avec un débit d'alimentation de 50,00 kg/h. Sa teneur en vanilline est de 0,9461 kg/kg.

La colonne 2 utilisée présente les caractéristiques et les conditions de fonctionnement suivantes :

**Tableau (IX)**

| **Colonne 2** | |
|---|---|
| Diamètre (mm) | 200 |
| Hauteur (mm) | 2500 |
| NET (nombre étages théoriques) total | 11 |
| NET (nombre étages théoriques) au dessous de l'alimentation | 9 |
| Pression en tête de colonne 2 (mm Hg) | 2 |
| Pression en pied de colonne 2 (mm Hg) | 9,5 |
| Température tête de colonne 2 (°C) | 120 |
| Température pied de colonne 2 (°C) | 162 |
| Facteur de charge (kg/m³)^{0,5} ×m.s⁻¹ | 3,9 |
| Volatilité relative moyenne (VA/lourds) | 8,1 |
| Reflux de tête de colonne 2 : *(F₉)* après condenseur 7 | 15,0 |
| Recyclage de pied de colonne 2 : *(F₁₂)* sur échangeur 8 | 400 |
| Charge thermique au bouilleur (kcal/h) | 7000 |

En tête de colonne 2, on récupère un flux gazeux *(F₈)* qui après condensation devient le flux *(F₁₀)* à raison de 49,0 kg/h et qui contient de la vanilline à une teneur de 0,9654 kg/kg.

En pied de colonne 2, on soutire un flux liquide *(F₁₁)* à raison de 404,9 kg/h et comprenant 0,531 kg de vanilline par kg de flux *(F₁₁)*.

Une fraction *(F₁₃)* du flux *(F₁₁)* alimente l'évaporateur raclé horizontal à pales fixes 16, l'autre fraction *(F₁₂)* étant réintroduit latéralement dans la colonne de distillation 2.

Les caractéristiques et les conditions de fonctionnement de l'évaporateur 16 sont rassemblées dans le tableau (X) :

**Tableau (X)**

| **Film raclé horizontal à pales fixes 16** | |
|---|---|
| Diametre du corps (mm) | 170 |
| Diamètre du rotor (mm) | 120 |
| Epaisseur de tube (mm) | 2 |
| Longueur (mm) | 500 |
| Surface échange totale paroi+rotor (m²) | 0,30 |
| Nature des pales fixes de raclage (acier AISI 316) | profilées |
| Vitesse de rotation du rotor (tours/mn) | 50 |
| Pression évaporateur (mm Hg) | 2 |
| Température caloporteur (°C) | 195 |
| Température moyenne de film (°C) | 183,5 |
| Viscosité moyenne de l'alimentation (Cp0) | 120 |
| Coef. de transfert de chaleur global (kcal/hm²°C) | 149 |
| Charge thermique de l'évaporateur à film raclé (kcal/h) | 496 |
| Volatilité relative moyenne (VA/lourds) | 8,0 |
| | |
| Alimentation depuis colonne 2 : *(F₁₃)* (kg/h) | 4,877 |
| Teneur en VA dans *(F₁₃)* (kg/kg) | 0,5310 |
| Vaporisation et recyclage à la colonne 2 : *(F₁₄)* (kg/h) | 3,866 |
| Teneur en VA dans *(F₁₄)* (kg/kg) | 0,6614 |
| Liquide en sortie d'évaporateur, goudrons extraits : *(F₁₅)* (kg/h) | 1,011 |
| Teneur en VA dans *(F₁₅)* (kg/kg) | 0,0320 |
| Pertes VA dans *(F₁₅)* (kg/h) | 0,032 |
| Taux de vaporisation de l'évaporateur à film raclé horizontal (% masse) | 79,3 |
| **Rendement de récupération évaporateur à film raclé horizontal en VA (% masse)** | 98,8 |

## Revendications

1. Procédé de séparation et de récupération d'un aldéhyde aromatique à partir d'un mélange comprenant un aldéhyde aromatique, des impuretés légères et lourdes, des goudrons **caractérisé par le fait que** l'on introduit le flux liquide comprenant ledit mélange dans un évaporateur, que l'on vaporise ledit mélange de telle sorte que l'on sépare d'une part un flux gazeux comprenant l'aldéhyde aromatique, les impuretés légères, les impuretés lourdes vaporisables à la température d'évaporation et de pression choisies et d'autre part un flux liquide comprenant essentiellement les goudrons et que l'on récupère l'aldéhyde aromatique à partir du flux gazeux séparé,
l'aldéhyde aromatique répondant à la formule suivante : 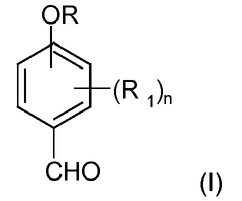 dans ladite formule :
- R représente un atome d'hydrogène ou un groupe alkyle,
- les groupes R₁, identiques ou différents, représentent un atome d'hydrogène, un groupe hydroxyle, un groupe alkyle ou alkoxy
- n est un nombre de 0 à 4,
une étape de concentration du mélange étant effectuée avant l'étape de séparation par évaporation, la concentration de l'aldéhyde aromatique dans le flux liquide après l'étape de concentration étant compris entre 10% et 60% en poids et celle des lourds entre 40% et 90% en poids.

2. Procédé selon la revendication 1 **caractérisé par le fait que** le mélange de départ comprend de 55 à 94 % en poids d'un aldéhyde aromatique, de 6 à 45% en poids des impuretés légères et lourdes, des goudrons

3. Procédé selon l'une des revendications 1 et 2 **caractérisé par le fait que** l'on recycle le flux gazeux séparé comprenant essentiellement l'aldéhyde aromatique obtenu, à l'état gazeux ou après condensation, dans une étape en amont de cette étape de séparation.

4. Procédé selon la revendication 1 **caractérisé par le fait que** l'on recycle le flux liquide séparé comprenant les goudrons, à l'entrée de l'évaporateur.

5. Procédé selon la revendication 2 **caractérisé par le fait que** le mélange comprenant l'aldéhyde aromatique est obtenu par extraction à l'aide d'un solvant organique, effectuée après introduction d'un groupe formyle.

6. Procédé selon la revendication 5 **caractérisé par le fait que** l'on effectue la séparation de l'aldéhyde aromatique du solvant d'extraction par distillation conçue pour obtenir :
- en pied de distillation, une phase liquide comprenant l'aldéhyde aromatique accompagné de différentes impuretés légères et des lourds,
- en tête de distillation, une phase gazeuse comprenant le solvant d'extraction et de l'aldéhyde aromatique à une faible concentration permettant le recyclage du solvant.

7. Procédé selon la revendication 6 **caractérisé par le fait que** la concentration est réalisée sur le flux liquide obtenu en pied de distillation avant de l'introduire dans l'évaporateur.

8. Procédé selon la revendication 7 **caractérisé par le fait que** l'on concentre le flux par distillation conçue pour obtenir :
- en pied de distillation, l'aldéhyde aromatique accompagné des lourds,
- en tête de distillation, une phase gazeuse comprenant essentiellement l'aldéhyde aromatique, les impuretés légères et des impuretés lourdes à une teneur inférieure à 100 ppm.

9. Procédé selon l'une des revendications 3, 7 et 8 **caractérisé par le fait que** l'on recycle le flux gazeux séparé comprenant essentiellement l'aldéhyde aromatique obtenu, à l'état gazeux ou après condensation, à l'étape de concentration par distillation.

10. Procédé selon l'une des revendications 1 et 2 **caractérisé par le fait que** l'on récupère l'aldéhyde aromatique à partir du mélange comprenant un aldéhyde aromatique, des impuretés légères et lourdes, des goudrons par vaporisation dans un évaporateur à film tombant ou à film ruisselant.

11. Procédé selon la revendication 10 **caractérisé par le fait que** le taux de vaporisation exprimé par le rapport pondéral entre le flux gazeux et le flux d'alimentation est compris entre 5 et 75 %, de préférence entre 5 et 50 %.

12. Procédé selon la revendication 10 **caractérisé par le fait que** l'on recycle le flux liquide séparé comprenant les goudrons avec un taux de recyclage de 50 à 75%.

13. Procédé selon l'une des revendications 1 et 2 **caractérisé par le fait que** l'on récupère l'aldéhyde aromatique à partir du mélange comprenant un aldéhyde aromatique, des impuretés légères et lourdes, des goudrons par vaporisation dans un évaporateur à film raclé.

14. Procédé selon la revendication 13 **caractérisé par le fait que** le taux de vaporisation exprimé par le rapport pondéral entre le flux gazeux et le flux d'alimentation est compris entre 20 et 90 %, de préférence entre 40 et 80 %.

15. Procédé selon la revendication 13 **caractérisé par le fait que** l'on recycle le flux liquide séparé comprenant les goudrons avec un taux de recyclage de 0 à 50 %.

16. Procédé selon l'une des revendications 1 et 2 **caractérisé par le fait que** l'on récupère l'aldéhyde aromatique à partir du mélange comprenant un aldéhyde aromatique, des impuretés légères et lourdes, des goudrons par vaporisation dans un évaporateur à court trajet à condenseur interne.

17. Procédé selon la revendication 16 **caractérisé par le fait que** le taux de vaporisation exprimé par le rapport pondéral entre le flux gazeux et le flux d'alimentation est compris entre 10 et 95 %, de préférence entre 15 et 90 %.

18. Procédé selon la revendication 16 **caractérisé par le fait que** l'on recycle le flux liquide séparé comprenant les goudrons avec un taux de recyclage de 0 à 50 %.

19. Procédé selon l'une des revendications 1 et 2 **caractérisé par le fait que** l'on récupère l'aldéhyde aromatique à partir du mélange comprenant un aldéhyde aromatique, des impuretés légères et lourdes, des goudrons par vaporisation dans un évaporateur raclé horizontal.

20. Procédé selon la revendication 19 **caractérisé par le fait que** le taux de vaporisation exprimé par le rapport pondéral entre le flux gazeux et le flux d'alimentation est compris entre 10 et 90 %, de préférence entre 15 et 90 %.

21. Procédé selon la revendication 19 **caractérisé par le fait que** l'on recycle le flux liquide séparé comprenant les goudrons avec un taux de recyclage de 0 à 50 %.

22. Procédé selon l'une des revendications 1 à 21 **caractérisé par le fait qu'**il est conduit sous atmosphère de gaz inertes, de préférence sous atmosphère d'azote.

23. Procédé selon l'une des revendications 1 à 22 **caractérisé par le fait que** l'aldéhyde aromatique est l'un des aldéhydes suivants :
- la vanilline,
- l'éthylvanilline (3-éthoxy-4-hydroxybenzaldéhyde),
- l'isovanilline (4-méthoxy-3-hydroxybenzaldéhyde),
- l'o-vanilline (3-méthoxy-2-hydroxybenzaldéhyde),
- l'aldéhyde protocatéchique,
- l'aldéhyde syringique (3,5-diméthoxy-4-hydroxybenzaldéhyde),
- l'aldéhyde vératrique (3,4-diméthoxybenzaldéhyde),
- le p-hydroxybenzaldéhyde,

24. Procédé selon la revendication 23 **caractérisé par le fait que** l'aldéhyde aromatique est la vanilline.

25. Procédé selon la revendication 23 **caractérisé par le fait que** l'aldéhyde aromatique est l'éthylvanilline.

## Patentansprüche

1. Verfahren zur Abtrennung und Gewinnung eines aromatischen Aldehyds aus einem Gemisch, das einen aromatischen Aldehyd, leichte und schwere Verunreinigungen und Teer umfasst, **dadurch gekennzeichnet, dass** man den flüssigen Strom, der das Gemisch umfasst, in einen Verdampfer einträgt, das Gemisch so verdampft, dass einerseits ein Gasstrom, der den aromatischen Aldehyd, die leichten Verunreinigungen und die schweren Verunreinigungen, die bei der gewählten Verdampfungstemperatur und dem gewählten Druck verdampfbar sind, umfasst, und andererseits ein flüssiger Strom, der im Wesentlichen den Teer umfasst, abgetrennt werden, und den aromatischen Aldehyd aus dem abgetrennten Gasstrom gewinnt,
wobei der aromatische Aldehyd der folgenden Formel entspricht: 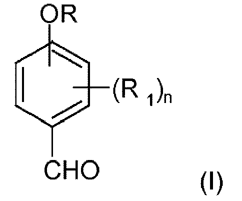 in welcher:
- R für ein Wasserstoffatom oder eine Alkylgruppe steht,
- die Gruppen R₁ gleich oder verschieden sind und für ein Wasserstoffatom, eine Hydroxylgruppe oder eine Alkyl- oder Alkoxygruppe stehen,
- n für eine Zahl von 0 bis 4 steht,
wobei vor dem Schritt des Abtrennens durch Verdampfung ein Schritt des Aufkonzentrierens des Gemischs durchgeführt wird, wobei die Konzentration des aromatischen Aldehyds in dem flüssigen Strom nach dem Schritt des Aufkonzentrierens zwischen 10 und 60 Gew.-% liegt und die Konzentration der schweren Verbindungen zwischen 40 und 90 Gew.-% liegt.

2. Verfahren nach Anspruch 1, **dadurch gekennzeichnet, dass** das Ausgangsgemisch 55 bis 94 Gew.-% eines aromatischen Aldehyds, 6 bis 45 Gew.-% leichte und schwere Verunreinigungen und Teer enthält.

3. Verfahren nach Anspruch 1 oder 2, **dadurch gekennzeichnet, dass** man den abgetrennten Gasstrom, der im Wesentlichen den erhaltenen aromatischen Aldehyd enthält, in gasförmigem Zustand oder nach Kondensation zu einem diesem Abtrennungschritt vorgeschalteten Schritt zurückführt.

4. Verfahren nach Anspruch 1, **dadurch gekennzeichnet, dass** man den abgetrennten flüssigen Strom, der den Teer enthält, zum Eingang des Verdampfers zurückführt.

5. Verfahren nach Anspruch 2, **dadurch gekennzeichnet, dass** man das den aromatischen Aldehyd enthaltende Gemisch durch Extraktion mit Hilfe eines organischen Lösungsmittels, die nach Einführung einer Formylgruppe durchgeführt wird, erhält.

6. Verfahren nach Anspruch 5, **dadurch gekennzeichnet, dass** man die Abtrennung des aromatischen Aldehyds von dem Extraktionslösungsmittel durch Destillation durchführt, welche so konzipiert ist, dass man Folgendes erhält:
- am Sumpf der Destillation eine flüssige Phase, die den aromatischen Aldehyd zusammen mit verschiedenen leichten Verunreinigungen und den Schwersiedern umfasst,
- am Kopf der Destillation eine Gasphase, die das Extraktionslösungsmittel und den aromatischen Aldehyd in einer geringen Konzentration, die die Rückführung des Lösungsmittels erlaubt, umfasst.

7. Verfahren nach Anspruch 6, **dadurch gekennzeichnet, dass** das Aufkonzentrieren an dem am Sumpf der Destillation erhaltenen flüssigen Strom vor dem Eintragen dieses Stroms in den Verdampfer durchgeführt wird.

8. Verfahren nach Anspruch 7, **dadurch gekennzeichnet, dass** man den Strom durch Destillation aufkonzentriert, welche so konzipiert ist, dass man Folgendes erhält:
- am Sumpf der Destillation den aromatischen Aldehyd zusammen mit den Schwersidern,
- am Kopf der Destillation eine Gasphase, die im Wesentlichen den aromatischen Aldehyd, die leichten Verunreinigungen und die schweren Verunreinigungen in einem Gehalt von weniger als 100 ppm umfasst.

9. Verfahren nach einem der Ansprüche 3, 7 und 8, **dadurch gekennzeichnet, dass** man den abgetrennten Gasstrom, der im Wesentlichen den erhaltenen aromatischen Aldehyd umfasst, in gasförmigem Zustand oder nach Kondensation zu dem Schritt des Aufkonzentrierens durch Destillation zurückführt.

10. Verfahren nach Anspruch 1 oder 2, **dadurch gekennzeichnet, dass** man den aromatischen Aldehyd aus dem Gemisch, das einen aromatischen Aldehyd, leichte und schwere Verunreinigungen und Teer umfasst, durch Verdampfung in einem Fallfilm- oder Rieselfilmverdampfer gewinnt.

11. Verfahren nach Anspruch 10, **dadurch gekennzeichnet, dass** die durch das Gewichtsverhältnis zwischen dem Gasstrom und dem Einsatzstrom ausgedrückte Verdampfungsrate zwischen 5 und 75% und vorzugsweise zwischen 5 und 50% liegt.

12. Verfahren nach Anspruch 10, **dadurch gekennzeichnet, dass** man den abgetrennten flüssigen Strom, der den Teer enthält, mit einer Rückführungsrate von 50 bis 75% zurückführt.

13. Verfahren nach Anspruch 1 oder 2, **dadurch gekennzeichnet, dass** man den aromatischen Aldehyd aus dem Gemisch, das einen aromatischen Aldehyd, leichte und schwere Verunreinigungen und Teer umfasst, durch Verdampfung in einem Wischfilmverdampfer gewinnt.

14. Verfahren nach Anspruch 13, **dadurch gekennzeichnet, dass** die durch das Gewichtsverhältnis zwischen dem Gasstrom und dem Einsatzstrom ausgedrückte Verdampfungsrate zwischen 20 und 90% und vorzugsweise zwischen 40 und 80% liegt.

15. Verfahren nach Anspruch 13, **dadurch gekennzeichnet, dass** man den abgetrennten flüssigen Strom, der den Teer enthält, mit einer Rückführungsrate von 0 bis 50% zurückführt.

16. Verfahren nach Anspruch 1 oder 2, **dadurch gekennzeichnet, dass** man den aromatischen Aldehyd aus dem Gemisch, das einen aromatischen Aldehyd, leichte und schwere Verunreinigungen und Teer umfasst, durch Verdampfung in einem Kurzwegverdampfer mit internem Kondensator gewinnt.

17. Verfahren nach Anspruch 16, **dadurch gekennzeichnet, dass** die durch das Gewichtsverhältnis zwischen dem Gasstrom und dem Einsatzstrom ausgedrückte Verdampfungsrate zwischen 10 und 95% und vorzugsweise zwischen 15 und 90% liegt.

18. Verfahren nach Anspruch 16, **dadurch gekennzeichnet, dass** man den abgetrennten flüssigen Strom, der den Teer enthält, mit einer Rückführungsrate von 0 bis 50% zurückführt.

19. Verfahren nach Anspruch 1 oder 2, **dadurch gekennzeichnet, dass** man den aromatischen Aldehyd aus dem Gemisch, das einen aromatischen Aldehyd, leichte und schwere Verunreinigungen und Teer umfasst, durch Verdampfung in einem horizontalen Wischverdampfer gewinnt.

20. Verfahren nach Anspruch 19, **dadurch gekennzeichnet, dass** die durch das Gewichtsverhältnis zwischen dem Gasstrom und dem Einsatzstrom ausgedrückte Verdampfungsrate zwischen 10 und 90% und vorzugsweise zwischen 15 und 90% liegt.

21. Verfahren nach Anspruch 19, **dadurch gekennzeichnet, dass** man den abgetrennten flüssigen Strom, der den Teer enthält, mit einer Rückführungsrate von 0 bis 50% zurückführt.

22. Verfahren nach einem der Ansprüche 1 bis 21, **dadurch gekennzeichnet, dass** es unter Inertgasatmosphäre, vorzugsweise unter Stickstoffatmosphäre, durch geführt wird.

23. Verfahren nach einem der Ansprüche 1 bis 22, **dadurch gekennzeichnet, dass** es sich bei dem aromatischen Aldehyd um einen der folgenden Aldehyde handelt:
- Vanillin,
- Ethylvanillin (3-Ethoxy-4-hydroxybenz-aldehyd),
- Isovanillin (4-Methoxy-3-hydroxybenzaldehyd),
- o-Vanillin (3-Methoxy-2-hydroxybenzaldehyd),
- Protocatechualdehyd,
- Syringaldehyd (3,5-Dimethoxy-4-hydroxy-benzaldehyd),
- Veratrumaldehyd (3,4-Dimethoxybenzaldehyd),
- p-Hydroxybenzaldehyd.

24. Verfahren nach Anspruch 23, **dadurch gekennzeichnet, dass** es sich bei dem aromatischen Aldehyd um Vanillin handelt.

25. Verfahren nach Anspruch 23, **dadurch gekennzeichnet, dass** es sich bei dem aromatischen Aldehyd um Ethylvanillin handelt.

## Claims

1. Process for separating and recovering an aromatic aldehyde from a mixture comprising an aromatic aldehyde, light and heavy impurities and tar, **characterized in that** the liquid stream comprising said mixture is introduced into an evaporator, said mixture is vaporized such that, on the one hand, a gas stream comprising the aromatic aldehyde, the light impurities, the heavy impurities that can be vaporized at the selected evaporation temperature and pressure, and, on the other hand, a liquid stream essentially comprising the tar are separated, and **in that** the aromatic aldehyde is recovered from the separated gas stream, the aromatic aldehyde corresponding to the following formula: 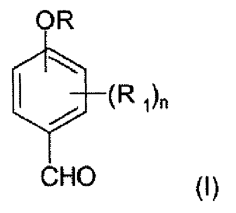 in said formula:
- R represents a hydrogen atom or an alkyl group,
- the R₁ groups, which may be identical or different, represent a hydrogen atom, a hydroxyl group, or an alkyl or alkoxy group,
- n is a number from 0 to 4,
a step of concentrating the mixture being carried out before the step of separation by evaporation, the concentration of the aromatic aldehyde in the liquid stream after the concentrating step being between 10% and 60% by weight, and that of the heavy compounds between 40% and 90% by weight.

2. Process according to Claim 1, **characterized in that** the starting mixture comprises from 55% to 94% by weight of an aromatic aldehyde, and from 6% to 45% by weight of light and heavy impurities, and tar.

3. Process according to either of Claims 1 and 2, **characterized in that** the separated gas stream essentially comprising the aromatic aldehyde obtained is recycled, in the gaseous state or after condensation, to a step upstream of this separation step.

4. Process according to Claim 1, **characterized in that** the separated liquid stream comprising tar is recycled to the input of the evaporator.

5. Process according to Claim 2, **characterized in that** the mixture comprising the aromatic aldehyde is obtained by extraction using an organic solvent, carried out after introduction of a formyl group.

6. Process according to Claim 5, **characterized in that** the separation of the aromatic aldehyde from the extraction solvent is carried out by distillation designed so as to obtain:
- at the distillation bottom, a liquid phase comprising the aromatic aldehyde accompanied by various light impurities and the heavy compounds,
- at the distillation top, a gas phase comprising the extraction solvent and aromatic aldehyde at a low concentration enabling recycling of the solvent.

7. Process according to Claim 6, **characterized in that** the concentrating is carried out on the liquid stream obtained at the distillation bottom before it is introduced into the evaporator.

8. Process according to Claim 7, **characterized in that** the stream is concentrated by distillation designed so as to obtain:
- at the distillation bottom, the aromatic aldehyde accompanied by the heavy compounds,
- at the distillation top, a gas phase essentially comprising the aromatic aldehyde, the light impurities and heavy impurities at a content of less than 100 ppm.

9. Process according to one of Claims 3, 7 and 8, **characterized in that** the separated gas stream essentially comprising the aromatic aldehyde obtained is recycled, in the gaseous state or after condensation, to the step of concentration by distillation.

10. Process according to either of Claims 1 and 2, **characterized in that** the aromatic aldehyde is recovered from the mixture comprising an aromatic aldehyde, light and heavy impurities, and tar, by vaporization in a falling-film evaporator.

11. Process according to Claim 10, **characterized in that** the vaporization rate, expressed by the weight ratio between the gas stream and the feed stream, is between 5% and 75%, preferably between 5% and 50%.

12. Process according to Claim 10, **characterized in that** the separated liquid stream comprising the tar is recycled with a recycling rate of 50% to 75%.

13. Process according to either of Claims 1 and 2, **characterized in that** the aromatic aldehyde is recovered from the mixture comprising an aromatic aldehyde, light and heavy impurities, and tar by vaporization in a wiped-film evaporator.

14. Process according to Claim 13, **characterized in that** the vaporization rate, expressed by the weight ratio between the gas stream and the feed stream, is between 20% and 90%, preferably between 40% and 80%.

15. Process according to Claim 13, **characterized in that** the separated liquid stream comprising the tar is recycled with a recycling rate of 0% to 50%.

16. Process according to either of Claims 1 and 2, **characterized in that** the aromatic aldehyde is recovered from the mixture comprising an aromatic aldehyde, light and heavy impurities, and tar by vaporization in a short-path evaporator with an internal condenser.

17. Process according to Claim 16, **characterized in that** the vaporization rate expressed by the weight ratio between the gas stream and the feed stream is between 10% and 95%, preferably between 15% and 90%.

18. Process according to Claim 16, **characterized in that** the separated liquid stream comprising the tar is recycled with a recycling rate of 0% to 50%.

19. Process according to either of Claims 1 and 2, **characterized in that** the aromatic aldehyde is recovered from the mixture comprising an aromatic aldehyde, light and heavy impurities, and tar by vaporization in a horizontal wiped evaporator.

20. Process according to Claim 19, **characterized in that** the vaporization rate, expressed by the weight ratio between the gas stream and the feed stream, is between 10% and 90%, preferably between 15% and 90%.

21. Process according to Claim 19, **characterized in that** the separated liquid stream comprising the tar is recycled with a recycling rate of 0% to 50%.

22. Process according to one of Claims 1 to 21, **characterized in that** it is carried out under an inert gas atmosphere, preferably under a nitrogen atmosphere.

23. Process according to one of Claims 1 to 22, **characterized in that** the aromatic aldehyde is one of the following aldehydes:
- vanillin,
- ethylvanillin (3-ethoxy-4-hydroxybenz-aldehyde),
- isovanillin (4-methoxy-3-hydroxybenz-aldehyde),
- o-vanillin (3-methoxy-2-hydroxybenzaldehyde),
- protocatechualdehyde,
- syringic aldehyde (3,5-dimethoxy-4-hydroxy-benzaldehyde),
- veratric aldehyde (3,4-dimethoxy-benzaldehyde),
- p-hydroxybenzaldehyde.

24. Process according to Claim 23, **characterized in that** the aromatic aldehyde is vanillin.

25. Process according to Claim 23, **characterized in that** the aromatic aldehyde is ethylvanillin.
